(19) Europäisches Patentamt
European Patent Office
Office européen des brevets

(11) Veröffentlichungsnummer: **0 045 277**
**B1**

(12) # EUROPÄISCHE PATENTSCHRIFT

(45) Veröffentlichungstag der Patentschrift: **02.10.85**

(51) Int. Cl.⁴: **C 07 D 213/22**, C 08 F 26/06

(21) Anmeldenummer: **81810284.0**

(22) Anmeldetag: **13.07.81**

(54) **Vinyl-substituierte 2,2'-Bipyridinverbindungen, daraus herstellbare Polymere, Verfahren zu deren Herstellung und Verwendung der Polymeren.**

(30) Priorität: **17.07.80 CH 5482/80**

(43) Veröffentlichungstag der Anmeldung: **03.02.82 Patentblatt 82/5**

(45) Bekanntmachung des Hinweises auf die Patenterteilung: **02.10.85 Patentblatt 85/40**

(84) Benannte Vertragsstaaten: **CH DE FR GB IT LI**

(56) Entgegenhaltungen:
**DE - A - 2 037 412**

**JOURNAL OF THE AMERICAN CHEMICAL SOCIETY, Band 102, 1980, Seiten 5543-5549 Columbus, Ohio, U.S.A. P.K. GHOSH et al.: "Photoelectrochemistry of Tris (bipyridyl) ruthenium (II) Covalently Attached to n-Type Sn02"**
**Polymer Reprints Japan, 29, 2, 280 (1980)**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Kaschig, Jürgen, Dr., Rötebuckweg 30, D-7800 Freiburg (DE)**
Erfinder: **Lohmann, Dieter, Dr., Mittelweg 56, CH-4142 Münchenstein (CH)**

**EP 0 045 277 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue vinyl-substituierte 2,2'-Bipyridinverbindungen, daraus herstellbare neue Polymere und Verfahren zu deren Herstellung.

In der US-Patentschrift 3 810 888 und den deutschen Offenlegungsschriften 2 037 412 und 2 049 057 sind komplexbildende Polymere beschrieben, die seitenständig oder in der Kette Bipyridinreste aufweisen können. Von der allgemeinen Definition werden sowohl Polymerisations- als auch Polykondensationsprodukte umfaßt. Die konkrete Offenbarung beschränkt sich jedoch auf die Herstellung von Polykondensationsprodukten. Die genannten Polymeren eignen sich zum Binden von Metallionen bei verschiedenen Anwendungen. Die daraus herstellbaren Polymeren können als Katalysatoren, z. B. für Hydrierungs-, Dehydrierungs- oder Isomerisierungsreaktionen, oder zum Überziehen von Metallen eingesetzt werden. Ferner ist bekannt, daß sich Übergangsmetallkomplexe, wie Metallkomplexe von Poly(styryl)-bipyridinen, z. B. Komplexe mit Pd(O), Palladiumacetat oder Wolframtetracarbonyl, Polystyrol-Tris(bi pyridyl)-Ruthenium(II)- und Bis(bipyridin)-poly-4-vinylpyridin-Ruthenium(II)-Komplexe, als Katalysatoren für verschiedene Reaktionen eignen, vor allem als Hydrierungs- oder Isomerisierungskatalysatoren oder für photochemische Reaktionen [vgl. z.B. Inorganic Chemistry, 17, No. 9, 2345 (1978); J. Org. Chem., 44, 1095 (1979) und 43, 2958 (1978); J. Am. Chem. Soc. 100:21, 6635 (1978), Inorganica Chimica Acta, 33, L 139 (1979) und Inorg. Chim. Acta, 44, L 289 (1980)]. Schließlich ist Polymer Preprints, Japan, 29, 2, 280 (1980) zu entnehmen, daß Polymere aus 6-Vinyl-2,2'-bipyridin Komplexe mit Übergangsmetallen bilden können.

Gegenstand der Erfindung sind neue 2,2'-Bipyridinverbindungen der Formel I

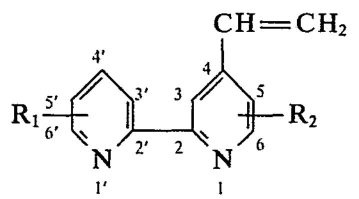

(I)

und deren Komplexe mit Metallen oder Metallverbindungen, die keine Alkalimetalle oder Erdalkalimetalle bzw. keine Alkalimetall- oder Erdalkalimetallverbindungen sind, worin $R_1$ Wasserstoff, $C_{1-6}$-Alkyl, $C_{1-6}$-Alkoxy, Phenyl oder Phenoxy und $R_2$ Wasserstoff oder Methyl bedeuten.

Alkyl- und Alkoxygruppen $R_1$ können geradkettig oder verzweigt sein, sind aber bevorzugt geradkettig und weisen 1—4 C-Atome auf. Als Beispiele geeigneter Alkyl- und Alkoxygruppen seien genannt: die Methyl-, Äthyl-, n-Propyl-, Isopropyl-, n-, sek- und tert.-Butyl-, n-Pentyl-, 2-Pentyl-, n-Hexyl-, Methoxy-, Äthoxy-, n-Propoxy-, Isopropoxy-, n-Butoxy, n-Pentyloxy- und n-Hexyloxygruppe.

Bevorzugt sind Verbindungen der Formel I und deren Komplexe, worin $R_1$ Wasserstoff oder in 4'-, 5'-, oder 6'-Stellung gebundenes $C_{1-4}$-Alkyl und $R_2$ Wasserstoff oder Methyl darstellen. Besonders bevorzugt sind Verbindungen der Formel I und deren Komplexe, worin $R_1$ in 4'- oder 6'-Stellung gebundenes Methyl und $R_2$ Wasserstoff oder $R_1$ Wasserstoff und $R_2$ in 6-Stellung gebundenes Methyl darstellen. Ganz besonders bevorzugt sind das 6-Methyl-4-vinyl-2,2'-bipyridin und dessen definitionsgemäße Komplexe.

Als Metalle zur Bildung von Komplexen mit Verbindungen der Formel I kommen z.B. solche der Hauptgruppen IIIa und IVa sowie die Nebengruppen IVb, Vb, VIb, VIIb, VIII, Ib und IIb des Periodischen Systems in Betracht. Geeignete Metallverbindungen zur Herstellung definitionsgemäßer Komplexe bzw. in definitionsgemäßen Komplexen sind vor allem neutrale oder ionische Metallverbindungen der vorgenannten Haupt- und Nebengruppen des Periodischen Systems, z. B. Salze oder Säuren, wobei bei den Salzen das Metall sowohl im Anion als auch im Kation vorliegen kann. Das Metallatom des Komplexes bzw. der zu dessen Herstellung verwendeten Metallverbindungen kann gegebenenfalls zusätzlich noch andere koordinative, kovalente oder ionische Bindungen aufweisen, die es mit anderen Ionen, Atomen oder Molekülen, z. B. einer oder mehreren weiteren Verbindungen der Formel I oder einem 2,2'-Bipyridinrest, verbinden.

Als Salze kommen sowohl Salze mit anorganischen als auch organischen Säuren in Betracht, wie Halogenide, besondere Chloride, Nitrate, Sulfate, Phosphate, Perchlorate und Carboxylate, wie Formiate, Acetate, Propionate und Stearate; ferner Salze, die ein komplexes Anion oder Kation aufweisen, z. B. Oxoderivate von Titan, Vanadium, Zirkon, Molybdän, Hafnium, Niob, Tantal, Wolfram und Uran; anionische Metallkomplexe von Halogenid-, Cyanid-, Thiocyanat-, Thiosulfat- und Orthophosphationen, wie Tetrachloroplatinat, Tetrachloropalladat oder Hexathiocyanatochromat. Als Beispiele derartiger Salze bzw. Komplexe seien genannt: Stannylchlorid, Bleiacetat; Kupfer(I)- oder -(II)chlorid, -bromid oder -jodid, Kupfer(II)acetat, -nitrat oder -sulfat, Kupfer(I)cyanid, Tetraacetonitrilokupfer(I)perchlorat, Silbernitrat; Zinkchlorid, -cyanid und -rhodanid, Cadmiumchlorid, -cyanid und -rhodanid, Quecksilberjodid oder -cyanid; Zirkoniumtetrachlorid; Vanadium(III)chlorid, Vanadiumoxidsulfat, Ammoniummetavanadat, Niob(V)chlorid, Tantal(V)chlorid, Urantetrachlorid oder -bromid, Uranylnitrat und -acetat; Chromcarbonyl, Chrom(III)chlorid, Hexathiocyanatochromat, Molybdänoxitrichlorid, Molybdäncarbonyl, Wolframoxitrichlorid, Wolframcarbonyl; Mangan(II)chlorid und -jodid; Eisen(III)nitrat, -phosphat, -sulfat oder -acetat, Eisen(II)- oder -(III)chlorid, Ruthenium(III)chlorid, Kaliumpentachlorohydroxyruthenat(IV), Dichloro-bis-(2,2'-bipyridin)ruthenium(II), Kobalt(II)chlorid, Kobalt(II)acetat, -nitrat oder -sulfat, Rhodium(II)acetat, Rhodium(III)chlorid, Kaliumrhodiumchlorid, Nickel(II)acetat, Nickel(II)-bromid oder -chlorid, Nickel(II)sulfat, Palladium(II)chlorid oder -jodid, Palladium(IV)chlorid, Palladiumacetat, Palladiumnitrat, Kaliumtetrachloropalladat, Kaliumtetrachloroplatinat, Kaliumhexachloroplatinat.

Bevorzugt sind Komplexe mit Metallen und Metallverbindungen der Nebengruppen Ib, IIb, IVb, Vb, VIb, VIIb und VIII, vor allem Metalle und Metallverbindungen der Nebengruppen Ib und VIII. Besonders bevorzugt sind Verbindungen der Formel I, die Eisen, Ruthenium, Kobalt, Nickel, Palladium, Platin oder Kupfer, vor allem Ruthenium, Palladium, Platin oder Kupfer, als komplexiertes Zentralatom enthalten. Ganz besonders bevorzugt sind Komplexe mit Palladium.

Als Säuren kommen z. B. Säuren, die den zuvor genannten Salzen mit einem komplexen Anion entsprechen, in Betracht, wie $H_2PtCl_6$ oder $H_2PdCl_4$.

Werden für die Herstellung von erfindungsgemäßen 2,2'-Bipyridinkomplexen Metallkomplexe eingesetzt, so werden Metallkomplexe mit mindestens zwei leicht ersetzbaren Liganden, welche Ligandenaustausch eingehen können, bevorzugt. Die Wertigkeit des mit der Verbindung der Formel I komplexierten Metalls wird durch die Art der für die Komplexherstellung verwendeten Metallverbindungen oder durch eine Oxidations- bzw. Reduktionsreaktion während oder nach der Komplexbildung bestimmt.

Die Verbindungen der Formel I können z. B. dadurch hergestellt werden, daß man eine Verbindung der Formel II

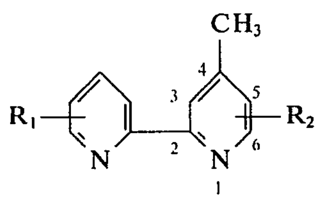

(II)

zu einer Verbindung der Formel IIIa oder IIIb

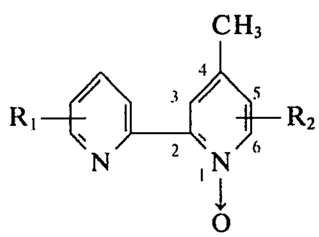

oder

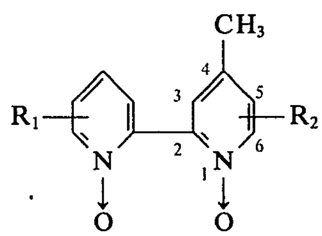

(IIIa) (IIIb)

oxidiert, die Verbindung der Formel IIIa oder IIIb mit einer Verbindung der Formel IV

$$(R'CO)_2O \qquad (IV)$$

zu einer Verbindung der Formel Va oder Vb

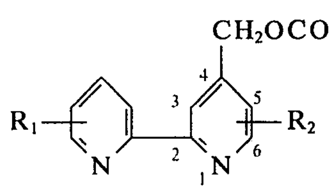

oder

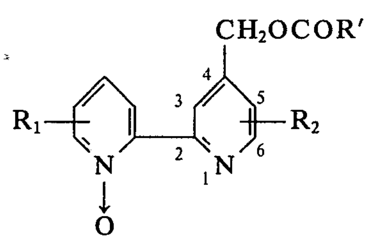

(Va) (Vb)

umsetzt, die Verbindung der Formel Vb in eine Verbindung der Formel Va überführt, die Verbindung der Formel Va in Gegenwart einer Base zu einer Verbindung der Formel VI

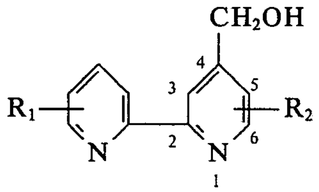

(VI)

verseift, die Verbindung der Formel VI durch Behandlung mit HCl oder HBr in die entsprechende Chlor- oder Brommethylverbindung der Formel VII

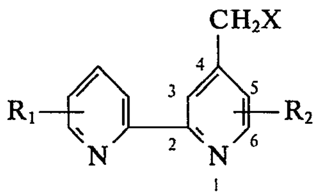

(VII)

überführt, die Verbindung der Formel VII mit einer Verbindung der Formel VIII

$P(R'')_3$ (VIII)

zu einem Phosphoniumsalz der Formel IX

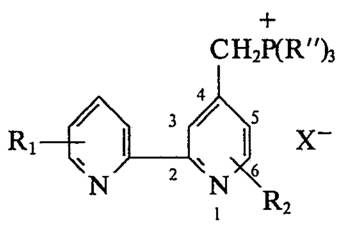

(IX)

umsetzt und das Phosphoniumsalz der Formel IX in Gegenwart einer Base mit Formaldehyd zu einer Verbindung der Formel I umsetzt. Dabei haben $R_1$ und $R_2$ die unter Formel I angegebene Bedeutung, X bedeutet Chlor oder Brom, R' stellt $C_{1-5}$-Alkyl und bevorzugt Methyl dar und die R'' bedeuten unabhängig voneinander $C_{1-5}$-Alkyl oder unsubstituiertes oder durch $C_{1-4}$-Alkyl oder $C_{1-4}$-Alkoxy mono- oder disubstituiertes Phenyl, bevorzugt je unsubstituiertes Phenyl.

Bevorzugt verwendet man als Verbindung der Formel VIII Triphenylphosphin. Anschließend können die Verbindungen der Formel I gewünschtenfalls auf an sich bekannte Weise in definitionsgemäße Komplexe übergeführt werden.

Für die Oxidation der Verbindungen der Formel II zu Verbindungen der Formel IIIa oder IIIb können an sich beliebige Oxidationsmittel eingesetzt werden, die keinen beeinträchtigenden Einfluß auf die übrigen Bestandteile des Moleküls ausüben. Geeignete Oxidationsmittel für die obige Reaktion sind z. B. Persäuren und organische Peroxide und Hydroperoxide. Besonders bevorzugt sind Persäuren, wie Peressigsäure, Perpropionsäure, Perbenzoesäure, Chlorperbenzoesäure, Monoperphthalsäuren oder Peroxide, wie Acetaldehyd-Monoperacetat und Dibenzoylperoxid. Die Oxidation wird mit Vorteil in Gegenwart eines inerten Lösungsmittels durchgeführt. Geeignete Lösungsmittel sind z. B. Essigsäureäthylester, Wasser, Essigsäure, Methylenchlorid, Aceton und Chloroform. Die Umsetzung der Verbindungen der Formel IIIa und IIIb mit den Anhydriden der Formel IV, vorzugsweise Acetanhydrid, kann in überschüssigem Reagens (Anhydrid der Formel IV) als Lösungsmittel oder bevorzugt in Gegenwart von chlorierten aliphatischen Kohlenwasserstoffen, wie Chloroform, Dichlormethan oder Dichloräthan, vorgenommen werden. Vor der weiteren Umsetzung zu Verbindungen der Formel VI werden allfällig noch vorhandene N-Oxidgruppen reduziert, z. B. durch Behandlung von Verbindungen der Formel Vb mit Phosphortrihalogeniden, Triphenylphosphit oder Triphenylphosphin. Die Verseifung der Verbindungen der Formel Va in Gegenwart einer Base zu Verbindungen der Formel VI wird mit Vorteil in wäßrigorganischem Medium, bevorzugt in wäßrig-alkoholischem Medium, durchgeführt. Als Basen verwendet man mit Vorteil Alkalimetall- oder Erdalkalimetallhydroxide, bevorzugt NaOH oder KOH. Die Behandlung der Hydroxymethylverbindungen der Formel VI mit HCl oder HBr erfolgt zweckmäßig bei Rückflußtemperatur und unter Verwendung konzentrierter wäßriger Chlor- oder Bromwasserstoffsäure. Die Verbindungen der Formel VII werden im allgemeinen ohne Zwischenisolierung direkt weiterverwendet.

Die Umsetzung der Verbindungen der Formel VII mit den Phosphinen der Formel VIII wird mit Vorteil in Gegenwart eines inerten organischen Lösungsmittels, wie Toluol, Tetrahydrofuran oder N,N-Dimethylformamid, und bei Temperaturen zwischen etwa 50 und 150°C vorgenommen. Die Umsetzung der Phosphoniumsalze mit Formaldehyd zu Verbindungen der Formel I wird in Gegenwart einer Base und gegebenenfalls in Gegenwart eines Lösungsmittels, sowie eines Phasentransferkatalysators und

eines Polymerisationsinhibitors durchgeführt. Als Basen eignen sich z. B. Natriumhydrid, n-Butyllithium, Alkalimetall- und Erdalkalimetallalkoholate, Alkalimetall- und Erdalkalimetallhydroxide, Alkalimetall- und Erdalkalimetallcarbonate sowie Trialkylamine mit je 2—4 C-Atomen in den Alkylgruppen. Bei der Reaktion verwendet man vorzugsweise ein inertes Lösungsmittel, wie Benzol, Toluol, Tetrahydrofuran, Dioxan, N,N-Dimethylformamid, Methylenchlorid, Methanol, Äthanol oder Wasser. Bevorzugte Lösungsmittel sind Wasser und besonders Gemische aus Wasser und einem wasserunlöslichen organischen Lösungsmittel, wie Methylenchlorid. Als Basen finden bevorzugt Alkalimetallhydroxide und -carbonate Verwendung. Als Phasentransferkatalysatoren eignen sich beispielsweise Kronenäther, Kryptanten und Tetraalkylammoniumsalze, wie Tetra-n-butylammoniumhydrogensulfat und -cyanid. Polymerisationsinhibitoren sind z. B. Dinitrochloraniline, Phenothiazin-Derivate, Diarylamine, Schwefel, Pikrinsäure, $\alpha$-Nitroso-$\beta$-naphthol, Hydrochinon und Phenole, wie Di-tert.butyl-p-kresol.

Verbindungen der Formel I können nach einem neuen Verfahren auch dadurch hergestellt werden, daß man entweder

a) eine Verbindung der Formel II

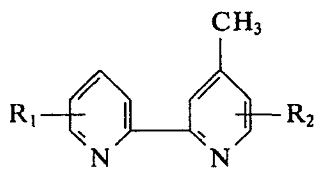

(II)

vorerst mit einem Metallierungsreagens behandelt und dann mit Formaldehyd in eine Verbindung der Formel X

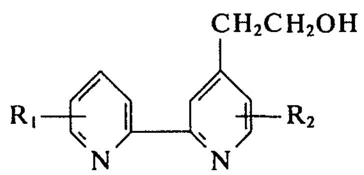

(X)

überführt oder

b) eine Verbindung der Formel IIa vorerst mit einem Metallierungsreagens behandelt und dann mit einer Verbindung der Formel XI

$$HalCH_2OCH_2R \qquad (XI)$$

zu einer Verbindung der Formel XII

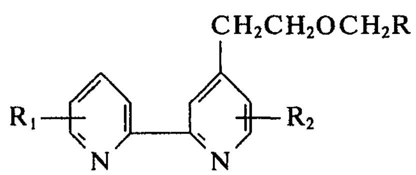

(XII)

umsetzt, die Verbindung der Formel XII zu einer Verbindung der Formel X hydriert und die Verbindung der Formel X zu einer Verbindung der Formel Ia dehydratisiert. Dabei gilt in den obigen Formeln für $R_1$ und $R_2$ das unter Formel I Angegebene, Hal stellt ein Halogenatom, besonders Brom oder Chlor, dar und R bedeutet unsubstituiertes oder durch $C_{1-6}$-Alkyl oder $C_{1-6}$-Alkoxy substituiertes Phenyl, vorzugsweise unsubstituiertes Phenyl. Verbindungen der Formel Ia können auch dadurch erhalten werden, daß man Verbindungen der Formel IIa wie unter b) beschrieben mit Metallierungsreagens und Verbindungen der Formel XI behandelt und das Reaktionsgemisch anschließend ohne Isolierung der Verbindungen der Formel XII erhitzt (thermische Eliminierung). Anschließend können die Verbindungen der Formel I gewünschtenfalls auf an sich bekannte Weise in definitionsgemäße Komplexe übergeführt werden.

Als Metallierungsreagens für die obigen Umsetzungen eignen sich vor allem Lithiumverbindungen, z. B. Alkyllithiumverbindungen, wie n-Butyllithium oder sec-Butyllithium, ferner Benzyllithium, Phenyllithium, Thienyllithium oder Lithiumalkylamide, wie Lithiumdiisopropylamid. Mit Vorteil wird Lithiumdiisopropylamid in Gegenwart von Hexamethylphosphorsäuretriamid eingesetzt. Die Reaktion wird zweckmäßig bei −70°C bis +80°C, bevorzugt bei 0 bis 25°C, in Gegenwart von inerten Lösungsmitteln, wie Diäthyläther oder Tetrahydrofuran, durchgeführt. Bevorzugtes Lösungsmittel ist Tetrahydrofuran. Die genannten bevorzugten Reaktionsbedingungen ermöglichen überraschenderweise die sehr selektive Umsetzung von Verbindungen der Formel II an der $CH_3$-Gruppe in 4-Stellung, ohne daß dabei andere Alkylgruppen am Pyridinring oder die Azomethingruppierung der Pyridinringe angegriffen werden. Die weitere Umsetzung mit Formaldehyd bzw. mit Verbindungen der Formel XI zu Verbindungen der Formel X bzw. XII wird zweckmäßig ohne Isolierung der erhaltenen metallierten Zwischenprodukte im gleichen Lösungsmittel vorgenommen.

Als Bipyridin-Verbindungen der Formel II können auch Isomerengemische, wie sie bei deren Herstellung anfallen, eingesetzt werden, z. B. Gemische aus 4,6-Dimethyl-2,2'-bipyridin, 3,6-Dimethyl-2,2'-bipyridin und 3,5-Dimethyl-2,2'-bipyridin, wobei jedoch nur Verbindungen mit einer Methylgruppe in 4-Stellung umgesetzt werden. Werden für die obigen Umsetzungen Isomerengemische eingesetzt, so ist die Verfahrensvariante b) bevorzugt. Die dabei anfallenden Verbindungen der Formel XII können leicht destillativ von nicht umgesetzten Ausgangsprodukten gereinigt werden.

Die Hydrierung der Verbindungen der Formel XII zu Verbindungen der Formel X wird bevorzugt in Gegenwart eines inerten organischen Lösungsmittels vorgenommen. Geeignete Lösungsmittel sind z. B. Äthanol, Methanol oder Dioxan, vor allem aber saure Medien, wie Äthanol/Chlorwasserstoffgemische, Essigsäure oder Trifluoressigsäure. Bevorzugt ist Trifluoressigsäure oder deren Gemisch mit Essigsäure. Die genannte Hydrierung wird mit Vorteil katalytisch durchgeführt. Als Katalysatoren eignen sich z. B. Kupferchromoxid und Nickelkatalysatoren, wie Raney-Nickel, besonders aber Edelmetall-Katalysatoren, wie Platin- und Palladiumkatalysatoren, ganz besonders Palladium-Kohlenstoff-Katalysatoren.

Die Dehydratisierung der Verbindungen der Formel X wird zweckmäßig in Gegenwart eines Alkalimetallhydroxides, wie Natrium- oder Kaliumhydroxid, durchgeführt. Vorteilhaft wird dabei ohne Lösungsmittel bei Temperaturen zwischen etwa 130 und 200°C gearbeitet. Bei der direkten Überführung von Verbindungen der Formel XII enthaltenden Reaktionsgemischen in Verbindungen der Formel I werden zweckmäßig Temperaturen von 120—200°C und Drucke von 133 bis 0,1 Pa verwendet.

Die allfällige Überführung der Verbindungen der Formel I in definitionsgemäße Komplexe erfolgt auf an sich bekannte Weise, z. B. indem man die Verbindungen der Formel I in ungelöster oder gelöster Form mit einer Lösung oder Suspension einer geeigneten Metallverbindung in Kontakt bringt. Geeignete Reaktionsmedien sind z. B. Wasser, N,N-Dimethylformamid, N,N-Dimethylacetamid, Acetonitril, Dioxan und Tetrahydrofuran. Als Metallverbindungen können z. B. Metallsalze oder Säuren der vorerwähnten Art, vor allem Halogenide oder Carboxylate oder aber Metallkomplexe mit mindestens zwei ersetzbaren koordinierten Liganden, die Ligandenaustausch bewirken können, verwendet werden, wie Tetraacetonitrilo-Kupfer(I)perchlorat, Dichloro-bis(2,2'-bipyridin)ruthenium(II) und Kaliumtetrachloroplatinat.

Die Verbindungen der Formeln II, IV, VIII und XI sind bekannt oder können nach an sich bekannten Methoden hergestellt werden. Verbindungen der Formel II können z. B. durch Umsetzung von gegebenenfalls substituierten Cyanpyridinen mit geeigneten Alkinen in Gegenwart von Kobalt-Katalysatoren erhalten werden [vgl. z. B. Synthesis, 600 (1975)].

Die Ausgangsstoffe bzw. Zwischenprodukte der Formeln IIIa, IIIb, Va, Vb, VI, VII, IX, X und XII, die speziell für die Herstellung der erfindungsgemäßen Verbindungen der Formel I entwickelt wurden, sind noch neu und in bezug auf R, R', R", $R_1$ und $R_2$ gelten die oben angegebenen Bevorzugungen.

Die Verbindungen der Formel I und deren erfindungsgemäße Komplexe stellen wertvolle Ausgangsmaterialien zur Herstellung von komplexbildenden oder komplexierten Polymeren dar.

Gegenstand der Erfindung sind daher auch neue, gegebenenfalls vernetzte Polymere, die dadurch erhältlich sind, daß man 2 bis 100 Mol% einer Verbindung der Formel I und/oder eines Komplexes einer Verbindung der Formel I mit Metallen oder Metallverbindungen, die keine Alkalimetalle oder Erdalkalimetalle bzw. keine Alkalimetall- oder Erdalkalimetallverbindungen sind, und 0 bis 98 Mol% einer Verbindung der Formel A)

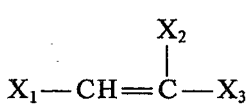

(A)

worin $X_1$ Wasserstoff, $X_2$ Wasserstoff, Chlor oder Methyl und $X_3$ Wasserstoff, Methyl, Chlor, —CN, —COOH, $-CONH_2$, Phenyl, Methylphenyl, Methoxyphenyl, Cyclohexyl, Pyridyl, Imidazolyl, Pyrrolidyl, —COO-Alkyl mit 1—12 C-Atomen im Alkyl, —COO-Phenyl,

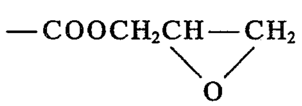

—COO-Alkyl-OH mit 1—4 C-Atomen im Alkyl, —OCO-Alkyl mit 1—4 C-Atomen im Alkyl, —OCO-Phenyl, —CO-Alkyl mit 1—3 C-Atomen im Alkyl, Alkoxy mit 1—20 C-Atomen oder Phenoxy oder $X_2$ Wasserstoff und $X_1$ und $X_3$ zusammen eine Anhydrid-Gruppierung, eine Gruppierung —CO—NR'''—CO— oder je —COOH oder —COOAlkyl mit 1—6 C-Atomen im Alkyl darstellen, wobei R''' geradkettiges oder verzweigtes $C_{1-18}$-Alkyl, Cyclohexyl oder Phenyl bedeutet, das durch $C_{1-6}$-Alkyl, Halogen, Cyano, Nitro und/oder $C_{1-3}$-Alkoxy mono- oder disubstituiert sein kann, in Gegenwart von 0 bis 60 Mol.% eines mehrfach ungesättigten Vernetzungsmittels polymerisiert und gegebenenfalls so erhaltene komplexbildende Polymere in Polymere überführt, die ganz oder teilweise mit Metallen oder Metallverbindungen komplexiert sind, die keine Alkalimetalle oder Erdalkalimetalle bzw. keine Alkalimetall- oder Erdalkalimetallverbindungen sind.

Werden dabei Komplexe von Verbindungen der Formel I verwendet, in denen das Metallatom, wie Kupfer, Eisen oder Ruthenium, mit weiteren Verbindungen der Formel I koordiniert ist, so entstehen – auch ohne Mitverwendung zusätzlicher Vernetzungsmittel – vernetzte Polymere.

Als Verbindungen der Formel A) werden solche bevorzugt, worin $X_1$ Wasserstoff, $X_2$ Wasserstoff oder Methyl und $X_3$ $-CONH_2$, Phenyl, Pyridyl, Pyrrolidyl, –COO-Alkyl–OH mit 2–4 C-Atomen im Alkyl oder –COO-Alkyl mit 1–12 C-Atomen im Alkylteil bedeuten. Ganz besonders bevorzugt sind Styrol, Methylacrylat, 2-Äthylhexylacrylat und 2-Hydroxyäthylmethacrylat.

Geeignete mehrfach ungesättigte Vernetzungsmittel sind z.B. Divinylbenzole, Divinylpyridine, Divinyltoluole, Divinylnaphthaline, Divinylxylole, Divinyläthylbenzole, Divinylsulfon, Divinylketon, Divinylsulfid, Divinylsebacat, Trivinylbenzole, Trivinylnaphthaline, Polyvinylanthracene; Äthylenglykoldiacrylat, Äthylenglykoldimethacrylat, Allylacrylat, Diallylphthalat, Diallylmaleat, Diallylfumarat, Diallylsuccinat, Diallylcarbonat, Diallylmalonat, Diallyloxalat, Diallyladipat, Diallylsebacat, Diallyltartrat, Diallylsilikat, Triallyltricarballylat, Triallylaconitrat, Triallylcitrat, Triallylphosphat, N,N'-Methylendiacrylamid, N,N'-Methylendimethylacrylamid, N,N'-Äthylendiacrylamid, Polyallyl- und Polyvinyläther von Äthylenglykol, Propantriol, Pentaerythrit, Resorcinol und den Monothio- oder Dithioderivaten von Äthylenglykol. Das Vernetzungsmittel wird vorzugsweise in einer Menge von 1 bis 30 Mol% eingesetzt. Bevorzugte Vernetzungsmittel sind Divinylpyridin und insbesondere Divinylbenzol. Durch geeignete Wahl der Comonomeren und/oder Vernetzungsmittel kann der Quellungsgrad der Polymeren den gewünschten spezifischen Anwendungen angepaßt werden.

Bevorzugt sind lineare Polymere mit einem durchschnittlichen Molekulargewicht von 1000 bis 5 000 000, die aus 3 bis 100 Mol% wiederkehrenden Strukturelementen der Formel B)

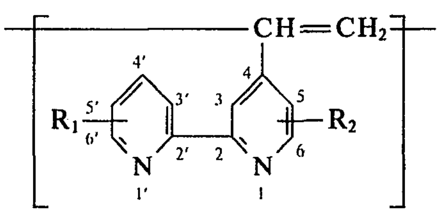

(B)

und/oder Komplexen derartiger Strukturelemente mit Metallen oder Metallverbinungen, die keine Alkalimetalle oder Erdalkalimetalle bzw. keine Alkalimetall- oder Erdalkalimetallverbindungen sind, wobei die Metallatome nicht mit weiteren Strukturelementen der Formel B) koordiniert sind, und 0 bis 97 Mol% wiederkehrenden Strukturelementen der Formel C)

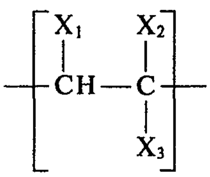

(C)

bestehen, worin für $R_1$ und $R_2$ das unter Formel I und für $X_1$, $X_2$ und $X_3$ das unter Formel A) Angegebene gilt, insbesondere derartige Polymere mit einem durchschnittlichen Molekulargewicht von 2000 bis 3 000 000 und/oder deren Komplexe, die aus 3 bis 100 Mol% wiederkehrenden Strukturelementen der Formel B) und/oder definitionsgemäßen Komplexen derartiger Strukturelemente der Formel B) und aus 0 bis 97 Mol% wiederkehrenden Strukturelementen der Formel C) bestehen, wobei die Gruppe $-CH-CH_2-$ in 4-Stellung gebunden ist, $R_1$ in 4'- oder 6'-Stellung gebundenes Methyl und $R_2$ Wasserstoff oder $R_1$ Wasserstoff und $R_2$ in 6-Stellung gebundenes Methyl darstellen, $X_1$ Wasserstoff, $X_2$ Wasserstoff oder Methyl und $X_3$ $-CONH_2$, Phenyl, Pyridyl, Pyrrolidyl, –COO-Alkyl–OH mit 2–4 C-Atomen im Alkyl oder –COO-Alkyl mit 1–12 C-Atomen im Alkyl bedeuten und wobei 5 bis 100 Prozent der Strukturelemente der Formel B) mit Metallen oder Metallverbindungen komplexiert sind, die keine Alkalimetalle oder Erdalkalimetalle bzw. keine Alkalimetall- oder Erdalkalimetallverbindungen sind. Besonders bevorzugt bedeuten $X_1$ Wasserstoff, $X_2$ Wasserstoff oder Methyl und $X_3$ Phenyl, $-COOCH_2CH_2OH$, $-COOCH_3$ oder

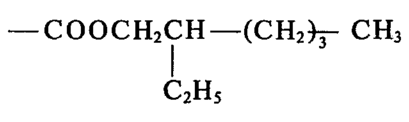

Bevorzugte komplexierende Metalle bzw. Metallverbindungen in vernetzten oder linearen erfindungsgemäßen Polymeren sind solche der unter Formel I erwähnten Art. Besonders bevorzugt sind vernetzte oder lineare Polymere, die ein Metall der Nebengruppen Ib oder VIII, besonders Eisen, Ruthenium, Kobalt, Nickel, Palladium, Platin oder Kupfer, vor allem Ruthenium, Palladium, Platin oder Kupfer und ganz besonders Palladium als komplexierte Zentralatome enthalten.

Die obigen linearen Polymeren können dadurch erhalten werden, daß man 3 bis 100 Mol% einer Verbindung der Formel I und/oder eines Komplexes einer Verbindung der Formel I mit Metallen oder Metallverbindungen, die keine Alkalimetalle oder Erdalkalimetalle bzw. keine Alkalimetall- oder Erdalkalimetallverbindungen sind, wobei das Metallatom nicht mit weiteren Verbindungen der Formel I koordiniert ist, mit 0 bis 97 Mol.% einer Verbindung der Formel A) polymerisiert und gegebenenfalls anschließend so erhaltene komplexbildende Polymere in Polymere überführt, die ganz oder teilweise mit Metallen oder Metallverbindungen komplexiert sind, die keine Alkalimetalle oder Erdalkalimetalle bzw. keine Alkalimetall- oder Erdalkalimetallverbindungen sind, wobei die Metallatome nicht mit weiteren Verbindungen der Formel I koordiniert sind.

Des weiteren sind vernetzte Polymere bevorzugt, die dadurch erhalten werden, daß man lineare Polymere mit einem durchschnittlichen Molekulargewicht von 1000 bis 5 000 000, die aus 3 bis 100 Mol.% wiederkehrenden Strukturelementen der Formel B) und 0 bis 97 Mol.% wiederkehrenden Strukturelementen der Formel C) bestehen, mit 5 bis 100 Mol.% Tetraacetonitrilo-Kupfer(I)perchlorat umsetzt. Als lineare Polymere werden dabei mit Vorteil solche der oben angegebenen bevorzugten Art eingesetzt.

Die allfällige Überführung der erfindungsgemäß erhaltenen Polymeren in ganz oder teilweise komplexierte Polymere kann analog wie im Vorangehenden für die Bipyridinverbindungen der Formel I beschrieben vorgenommen werden.

Die Polymerisation von Verbindungen der Formel I oder deren Komplexen mit Metallen oder Metallverbindungen, die keine Alkalimetalle oder Erdalkalimetalle bzw. keine Alkalimetall- oder Erdalkalimetallverbindungen sind, sowie deren Copolymerisation mit Verbindungen der Formel A), gegebenenfalls in Gegenwart von mehrfach ungesättigten Vernetzungsmitteln, kann auf an sich bekannte Weise vorgenommen werden, z. B. in Gegenwart üblicher anionischer Initiatoren. Bevorzugt ist die radikalische Polymerisation. Dabei verwendet man zweckmäßig etwa 0,01 bis 5 Gew.%, vorzugsweise 0,01 bis 1,5 Gew.%, bezogen auf das Gesamtgewicht der Monomeren und Vernetzungsmittel, an sich bekannter Radikalinitiatoren, wie anorganische oder organische Peroxide oder Azoverbindungen, z. B. Wasserstoffperoxid, Kaliumperoxidisulfat, tert-Butylhydroperoxid, Di-tert-butylperoxid, Peressigsäure, Dibenzoylperoxid, Diacylperoxide, Cumolhydroperoxid, tert-Butylperbenzoat, tert-Alkylperoxidicarbonate und $\alpha,\alpha'$-Azoisobutyronitril. Die Reaktionstemperaturen für die radikalische Polymerisation liegen im allgemeinen zwischen etwa 30 und 100°C. Die radikalische Polymerisation kann aber auch in der Kälte durchgeführt werden, wozu man auch Redoxsysteme in den oben genannten Konzentrationen verwenden kann, z. B. Gemische aus Peroxiden, wie Wasserstoffperoxid, und einem Reduktionsmittel, wie zweiwertige Eisenionen. Die Polymerisation kann in homogener Phase, z. B. in Substanz oder in Lösung, oder in heterogener Phase, d. h. als Fällungspolymerisation, Emulsions- oder Suspensionspolymerisation, erfolgen. Bevorzugt ist die Polymerisation in Lösung. Geeignete Lösungsmittel sind z. B. Toluol, N,N-Dimethylformamid, N,N-Dimethylacetamid und Acetonitril.

Erfindungsgemäß herstellbare, ganz oder teilweise komplexierte Polymere können als Katalysatoren, z. B. als Hydrierungskatalysatoren, wie zur Hydrierung von Alkenen oder Alkinen, als Isomerisierungskatalysatoren oder als Katalysatoren für die Acetoxylierung von Benzol eingesetzt werden. Definitionsgemäße Komplexe von Verbindungen der Formel I können ebenfalls als Katalysatoren verwendet werden. Vor allem wegen ihrer Unlöslichkeit in den üblichen Reaktionsmedien werden jedoch für diese Anwendung erfindungsgemäße komplexierte Polymere bevorzugt.

Erfindungsgemäße, mindestens teilweise mit Palladium oder einer Palladiumverbindung, besonders Palladiumacetat, komplexierte Polymere eignen sich mit guter Effizienz vor allem als Katalysatoren für Umvinylierungsreaktionen. Ihre Wirkung ist mit derjenigen homogener Katalysatoren, wie sie im allgemeinen für Umvinylierungsreaktionen verwendet werden, vergleichbar. Gegenüber vorbekannten Umvinylierungskatalysatoren, wie Kaliumtetrachloropalladat, haben sie den Vorteil, daß sie ohne Wiederaufbereitung direkt für weitere Reaktionen verwendet werden können.

Erfindungsgemäße nicht-komplexierte Polymere können zur Herstellung von entsprechenden komplexierten Polymeren verwendet werden. Derartige komplexbildende Polymere — wie auch die Verbindungen der Formel I — finden aber auch Anwendung als Metallionen-Fänger, z. B.

- für die Extraktionen von Edelmetallen, Seltenen Erden und radioaktiven Elementen, wie Uran, aus ihren Erzen oder Mineralien,
- für die Trennung von radioaktivem Cäsium von anderen Metallen, oder die Trennung verschiedener Metalle (mit der Ausnahme von Alkalimetallen und Erdalkalimetallen),
- für die Gewinnung von Chromsalzen aus Gerbereiabwässern,
- für die Entmineralisierung von organischen Lösungsmitteln ohne Einbringen von Fremdionen zur Herstellung von dielektrischen Flüssigkeiten,
- für die Reinigung von Industrieabwässern zur Entfernung von unerwünschten Metallionen.

Nach dem Binden der Metallionen können die erhaltenen Komplexe wieder in komplexbildende Polymere bzw. komplexbildende Verbindungen der Formel I übergeführt werden, z. B. durch Elution mit starken Säuren oder mit komplexbildenden Mitteln, wie Äthylendiamin oder Äthylendiaminotetraessigsäure.

Mit den Verbindungen der Formel I und deren Komplexen lassen sich lineare Polymere mit praktisch beliebigem durchschnittlichem Molekulargewicht herstellen. Durch Verwendung von geeigneten Comonomeren und/oder Vernetzungsmitteln können sogenannte maßgeschneiderte Polymere hergestellt werden, d. h. Polymere mit den spezifischen Anwendungen angepaßter Zusammensetzung und Zahl von komplexbildenden oder komplexierten Bipyridineinheiten. Die mit den Verbindungen der Formel I und deren Komplexen herstellbaren, gegebenenfalls vernetzten Polymeren zeichnen sich zudem durch eine hohe Stabilität gegen thermischen oder chemischen Abbau aus.

Die folgenden Beispiele dienen der Erläuterung der Erfindung.

## Beispiel 1

### 4-Vinyl-6-methyl-2,2′-bipyridin

#### a) Herstellung von 4-[2-(Benzyloxy)äthyl]-6-methyl-2,2′-bipyridin

36,4 g (0,2 Mol) eines Isomerengemisches aus 4,6-Dimethyl-2,2′-bipyridin (45 Gew.%), 3,6-Dimethyl-2,2′-bipyridin (45 Gew.%) und 3,5-Dimethyl-2,2′-bipyridin (10 Gew.%) werden in 150 ml wasserfreiem Tetrahydrofuran (THF) gelöst. Bei 0°C wird innerhalb von 2 Stunden eine Lösung von Lithiumdiisopropylamid und Hexamethylphosphorsäuretriamid in THF/n-Hexan [Volumenverhältnis ca. 1 : 1; hergestellt aus 12,2 g (0,12 Mol) Diisopropylamin in 50 ml wasserfreiem THF, 60 ml 2-molarer n-Butyllithium-Lösung in n-Hexan sowie 25,9 g (0,12 Mol) wasserfreiem Hexamethylphosphorsäuretriamid] zugetropft. Anschließend wird 30 Minuten bei 0°C und 15 Minuten bei 23°C gerührt. Bei −75°C werden innerhalb von 30 Minuten 20,3 g (0,13 Mol) Benzylchlormethyläther zugetropft. Das Reaktionsgemisch wird langsam auf 23°C erwärmt, 1 Stunde bei dieser Temperatur gerührt und dann in 200 ml 10%ige Salzsäure gegeben. Die Lösung wird dreimal mit je 250 ml Essigsäureäthylester gewaschen. Die wäßrige Phase wird mit Kaliumcarbonat alkalisch gestellt, und das erhaltene Produkt wird viermal mit je 250 ml Essigsäureäthylester extrahiert. Die vereinigten Essigsäureäthylester-Phasen werden über Natriumsulfat getrocknet, und das Lösungsmittel wird im Vakuum entfernt. Der Rückstand wird im Hochvakuum destilliert. Der Vorlauf (16,8 g; Kp. 75–83°C/0,27 Pa) enthält 78 Gew.% 3,6-Dimethyl-2,2′-bipyridin, 14 Gew.% 3,5-Dimethyl-2,2′-bipyridin sowie 3 Gew.% nicht umgesetztes 4,6-Dimethyl-2,2′-bipyridin. Bei 174–176°C/0,09 Pa destillieren 20,3 g 4-[2-(Benzyloxy)äthyl-6-methyl-2,2′-bipyridin; Ausbeute 78% d. Th., berechnet auf eingesetztes 4,6-Dimethyl-2,2′-bipyridin im Isomerengemisch.

$n_D^{20} = 1,6007$.

Analyse für $C_{20}H_{20}N_2O$:

| | | | |
|---|---|---|---|
| berechnet | C 78,92% | H 6,63% | N 9,20% |
| gefunden | C 78,70% | H 6,69% | N 9,40%. |

#### b) Herstellung von 4-(2-Hydroxyäthyl)-6-methyl-2,2′-bipyridin

##### Methode A

Eine Lösung von 22 g (0,072 Mol) 4-[2-(Benzyloxy)-äthyl]-6-methyl-2,2′-bipyridin in 110 ml Trifluoressigsäure wird mit 4,4 g Palladium/Kohlenstoff (5 Gew.% Pd) versetzt und bei 25°C hydriert. Nach 30 Minuten kommt die Wasserstoffaufnahme (1,78 l) zum Stillstand. Nach dem Abfiltrieren des Katalysators wird im Vakuum vom Lösungsmittel befreit, und der ölige Rückstand wird in 2 N Salzsäure gelöst. Die Lösung wird mit Essigsäureäthylester gewaschen und anschließend mit Kaliumcarbonat alkalisch gestellt (pH 9). Das erhaltene 4-(2-Hydroxyäthyl)-6-methyl-2,2′-bipyridin wird viermal mit je 150 ml Essigsäureäthylester extrahiert. Nach dem Waschen der vereinigten organischen Phasen mit gesättigter Kochsalzlösung und Trocknen über Natriumsulfat wird das Lösungsmittel im Vakuum abdestilliert. Der Rückstand kristallisiert nach mehrstündigem Stehen und kann aus n-Hexan/Essigsäureäthylester umkristallisiert werden. Ausbeute 13,5 g (87% d. Th.); Fp. 56°C.

Analyse für $C_{13}H_{14}N_2O$:

| | | | | |
|---|---|---|---|---|
| berechnet | C 72,88% | H 6,59% | N 13,08% | O 7,47% |
| gefunden | C 72,60% | H 6,80% | N 13,05% | O 7,46%. |

Methode B

16,6 g (0,0876 Mol) eines Isomerengemisches aus Dimethyl-2,2'-bipyridinen [Zusammensetzung wie oben unter a) angegeben] werden in 50 ml wasserfreiem THF gelöst. Bei 0°C wird eine Lösung von Lithiumdiisopropylamid und Hexamethylphosphorsäuretriamid in THF/n-Hexan [Volumenverhältnis ca. 1 : 1; hergestellt aus 10,1 g (0,1 Mol) Diisopropylamin in 50 ml wasserfreiem THF, 50 ml 2-molarer n-Butyllithium-Lösung in n-Hexan sowie 18 g (0,1 Mol) wasserfreiem Hexamethylphosphorsäuretriamid] zugetropft. Anschließend wird 30 Minuten bei 0°C und 30 Minuten bei 40°C gerührt. Bei 0 bis 10°C wird Formaldehyd-Gas [hergestellt durch Pyrolyse von Paraformaldehyd] eingeleitet, bis die Farbe von Schwarz auf Gelb umschlägt. Nach 5 Minuten Erhitzen auf 40°C werden unter Eiskühlung 250 ml verdünnte Salzsäure zugegeben. Nach dreimaligem Waschen mit je 300 ml Essigsäureäthylester wird mit Kaliumcarbonat alkalisch gestellt, und das Reaktionsprodukt wird dreimal mit je 350 ml Essigsäureäthylester extrahiert. Der Extrakt wird mit gesättigter Ammoniumsulfat-Lösung gewaschen, über Natriumsulfat getrocknet und im Vakuum vom Lösungsmittel befreit. Der Rückstand wird im Hochvakuum (4 Pa) auf maximal 130°C erhitzt, wobei neben Hexamethylphosphorsäuretriamid ein Gemisch aus 3,6-Dimethyl-2,2'-bipyridin (73 Gew.%), 3,5-Dimethyl-2,2'-bipyridin (3 Gew.%) sowie nicht umgesetztem 4,6-Dimethyl-2,2'-bipyridin (8 Gew.%) abdestilliert. Nach Chromatographie des Rückstandes (8,9 g) an Kieselgel (Elutionsmittel Toluol/Aceton, Vol.-Verhältnis 1 : 1) und Umkristallisieren aus n-Hexan/Essigsäureäthylester werden 4,2 g 4-(2-Hydroxyäthyl)-6-methyl-2,2'-bipyridin in kristalliner Form erhalten (50% d.Th.), berechnet auf eingesetztes 4,6-Dimethyl-2,2'-bipyridin im Isomerengemisch.

c) Herstellung von 4-Vinyl-6-methyl-2,2'-bipyridin

Methode A

7,6 g pulverförmiges Kaliumhydroxid, 5,4 g Aluminiumoxid-Perlen (»Shperalite SCS«® 9 3/6 mm der Fa. Rhône-Poulenc Ind.) sowie 0,2 g Hydrochinon werden in einer Destillationsblase auf 170°C erhitzt. Bei einem Druck von 6,7 Pa werden 10,9 g (0,051 Mol) geschmolzenes 4-(2-Hydroxyäthyl)-6-methyl-2,2'-bipyridin zugetropft. Das Reaktionsprodukt destilliert bei 100–105°C kontinuierlich ab. Nach der Redestillierung werden 8,7 g (87% d.Th.) 4-Vinyl-6-methyl-2,2'-bipyridin erhalten. Kp. 90–91°C/0,40 Pa. UV-Absorption (in Chloroform): $\lambda_{max} = 288$ mm ($\varepsilon = 10\,200$).

Analyse für $C_{13}H_{12}N_2$:
berechnet C 79,56% H 6,16% N 14,27%
gefunden C 79,36% H 6,19% N 14,38%.

Methode B

500 g (2,75 Mol) eines Isomerengemisches aus 4,6-Dimethyl-2,2'-bipyridin (45 Gew.%), 3,6-Dimethyl-2,2'-bipyridin (45 Gew.%) und 3,5-Dimethyl-2,2'-bipyridin (10 Gew.%) werden entsprechend dem Vorgehen gemäß Beispiel 1 (a) umgesetzt. Das entstandene Rohprodukt wird bei 133 bis 400 Pa 2 Stunden auf 120 bis 125°C, dann 45 Minuten auf 170°C (Badtemperatur) erhitzt. Dabei werden 299 g eines Destillats mit dem Siedebereich 65–107°C gewonnen, welches sich aus 65 Gew.% 3,6-Dimethyl-2,2'-bipyridin, 7 Gew.% 3,5-Dimethyl-2,2'-bipyridin sowie 17 Gew.% Benzylalkohol und 10 Gew.% Hexamethylphosphorsäuretriamid zusammensetzt. Der Rückstand wird bei 0,1 Pa erhitzt, wobei in einem Siedebereich von 48–170°C ein Gemisch abdestilliert, das nach Zugabe von Di-tert-butyl-p-kresol als Polymerisationsinhibitor einer fraktionierten Redestillation unterworfen wird. Dabei werden 51 g (4-(2-(Benzyloxy)äthyl)-6-methyl-2,2'-bipyridin, 11 g 3,6- und 3,5-Dimethyl-2,2'-bipyridin, 105 g Benzylalkohol und 147 g 4-Vinyl-6-methyl-2,2'-bipyridin (61 % d.Th.) erhalten.

Vergleichsbeispiel a

3-Methyl-6-vinyl-2,2'-bipyridin

a) Herstellung von 3,6-Dimethyl-2,2'-bipyridin-1,1'-dioxid

19 g (0,103 Mol) 3,6-Dimethyl-2,2'-bipyridin werden mit 17,3 g (0,227 Mol) wasserfreier Peressigsäure (als 2,28-molare Lösung in Essigsäureäthylester) 66 Stunden bei 23°C gerührt. Nach Zugabe von ca. 200 ml Äthylbenzol wird im Vakuum eingeengt, wiederum mit Äthylbenzol versetzt und erneut eingeengt. Der kristalline Rückstand wird aus Chlorbenzol umkristallisiert. Man erhält 18,9 g (85% d.Th.) 3,6-Dimethyl-2,2'-bipyridin-1,1'-dioxid; Fp. 225–230°C.

Analyse für $C_{12}H_{12}N_2O_2$:
berechnet C 66,66 % H 5,60 % N 12,96 % O 14,80 %
gefunden C 66,1 % H 5,6 % N 12,6 % O 15,1 %.

b) Herstellung von 3-Methyl-6-acetoxymethyl-2,2'-bipyridin-1'-oxid

Eine Lösung von 4 g (0,018 Mol) 3,6-Dimethyl-2,2'-bipyridin-1,1'-dioxid in 20 ml Chloroform wird bei 60°C und unter Stickstoffatmosphäre zu 20 ml Acetanhydrid getropft. Es wird 20 Stunden bei 60—65°C gerührt und anschließend im Vakuum eingeengt. Nach Zugabe von 50 ml Wasser sowie 50 ml Essigsäureäthylester wird mit Kaliumcarbonat alkalisch gestellt. Die Wasserphase wird abgetrennt und viermal mit je 50 ml Essigsäureäthylester/THF (Volumenverhälnis ca. 2 : 1) ausgewaschen. Die vereinigten organischen Phasen werden über Natriumsulfat getrocknet. Nach Abdestillieren des Lösungsmittels im Vakuum werden 4,44 g (96 % d. Th.) braunes Rohprodukt erhalten, welches bei 0°C zur Kristallisation gebracht werden kann. Das Rohprodukt kann direkt für weitere Umsetzungen eingesetzt werden. Nach Umkristallisieren aus Diäthyläther erhält man 73 % reines 3-Methyl-6-acetoxymethyl-2,2'-bi pyridin-1'-oxid; Fp. 105—107°C.

Analyse für $C_{14}H_{14}O_3N_2$:
berechnet C 65,11 % H 5,46 % N 10,85 % O 18,58 %
gefunden C 65,23 % H 5,48 % N 10,87 % O 18,43 %.

c) Herstellung von 3-Methyl-6-acetoxymethyl-2,2'-bipyridin

2,1 g (8,1 Mol) 3-Methyl-6-acetoxymethyl-2,2'-bipyridin-1'-oxid werden in 20 ml Benzol bei 45°C gelöst und unter Stickstoffatmosphäre tropfenweise mit 1,43 g (10,4 mMol) Phosphortrichlorid versetzt. Anschließend wird 30 Minuten unter Rückfluß gekocht. Nach dem Abkühlen auf 23°C wird mit 35 ml Wasser versetzt, und die Benzolphase wird abgetrennt. Die Wasserphase wird zweimal mit je 15 ml Essigsäureäthylester ausgewaschen. Die vereinigten organischen Phasen werden über Natriumsulfat getrocknet und im Vakuum eingeengt. Nach der Destillation im Kugelrohofen bei 125°C/0,13 Pa werden 1,51 g (77 % d. Th.) 3-Methyl-6-acetoxymethyl-2,2'-bipyridin in Form eines farblosen Öls erhalten.

Analyse für $C_{14}H_{14}N_2O_2$:
berechnet C 69,41 % H 5,83 % N 11,57 % O 13,21 %
gefunden C 68,93 % H 5,58 % N 11,54 % O 13,12 %.

d) Herstellung von 3-Methyl-6-hydroxymethyl-2,2'-bipyridin

1,4 g (5,8 mMol) 3-Methyl-6-acetoxymethyl-2,2'-bipyridin werden in einer Mischung aus 20 ml 10 %iger Natronlauge und 15 ml Äthanol 30 Minuten unter Rückfluß gekocht. Nach dem Abkühlen wird mit Salzsäure angesäuert und zweimal mit je 20 ml Essigsäureäthylester gewaschen. Die wäßrige Phase wird mit Kaliumcarbonat alkalisch gestellt, und das Reaktionsprodukt wird mit 40 ml Essigsäureäthylester extrahiert. Nach dem Trocknen über Natriumsulfat und dem Einengen im Vakuum wird das 3-Methyl-6-hydroxymethyl-2,2'-bipyridin aus Diäthyläther auskristallisiert. Ausbeute: 0,99 g (85 % d. Th.); Fp. 51—53°C. UV-Absorption (in Chloroform): $\lambda_{max} = 281$ mm ($\varepsilon = 9400$).

Analyse für $C_{12}H_{12}N_2O$:
berechnet C 71,98 % H 6,04 % N 13,99 % O 7,99 %
gefunden C 71,68 % H 5,97 % N 13,86 % O 8,00 %.

e) Herstellung von 3-Methyl-6-brommethyl-2,2'-bipyridin

0,9 g (4,5 mMol) 3-Methyl-6-hydroxymethyl-2,2'-bipyridin werden zusammen mit 5 ml 62 %iger Bromwasserstoffsäure 6 Stunden unter Rückfluß gekocht. Nach dem Abkühlen werden 10 ml Wasser sowie 20 ml Diäthyläther zugegeben. Die Wasserphase wird mit Kaliumcarbonat alkalisch gestellt, von der Ätherphase getrennt und mit weiteren 10 ml Diäthyläther gewaschen. Die vereinigten Ätherphasen werden über Natriumsulfat getrocknet und im Vakuum zur Trockne eingeengt (maximale Badtemperatur: 30°C). Es verbleiben 1,16 g (98 % d. Th.) 3-Methyl-6-brommethyl-2,2'-bipyridin als öliges Produkt, das sich bei Raumtemperatur unter Rotfärbung zersetzt und bei 0°C kristallisiert. Das 3-Methyl-6-brommethyl-2,2'-bipyridin wird unmittelbar weiterverwendet.

f) Herstellung von [3-Methyl-2,2'-bipyridin-6-yl-(methylen)]-triphenylphosphoniumbromid

1,16 g (4,4 mMol) 3-Methyl-6-brommethyl-2,2'-bipyridin werden zusammen mit 1,39 g (5,3 mMol) Triphenylphosphin in 50 ml N,N-Dimethylformamid 3 Stunden auf 80°C erhitzt. Bei 23°C werden unter schnellem Rühren 50 ml Diäthyläther zugegeben. Das ausfallende ölige Produkt kristallisiert nach kurzer Zeit und wird abgesaugt. Nach dem Umkristallisieren aus 25 ml Wasser werden 1,14 g (49 % d.Th.) [3-Methyl-2,2'-bipyridin-6-yl(methylen)]-triphenylphosphoniumbromid in Form farbloser Kristalle erhalten; Fp. 214—215°C.

Analyse für $C_{30}H_{26}BrN_2P \cdot 1\ H_2O$:

| | | | | | |
|---|---|---|---|---|---|
| berechnet | C 66,30% | H 5,19% | N 5,16% | Br 14,70% | P 5,70% |
| gefunden | C 66,61% | H 5,26% | N 5,23% | Br 14,98% | P 5,72%. |

g) Herstellung von 3-Methyl-6-vinyl-2,2'-bipyridin

In einer gut gerührten Emulsion aus 15 ml 35%iger Formaldehyd-Lösung, 20 ml Dichlormethan, 5 mg Di-tert-butyl-p-kresol sowie 22 mg Tetrabutylammoniumcyanid werden 0,9 g (1,7 mMol) [3-Methyl-2,2'-bipyridin-6-yl(methylen)]triphenylphosphoniumbromid-monohydrat gelöst. Bei 25°C wird eine Lösung aus 0,5 g Natriumhydroxid in 2,5 ml Wasser zugetropft. Nach 1 Stunde Rühren bei 23°C wird die Dichlormethanphase abgetrennt und mit 20 ml Wasser gewaschen. Nach Zugabe von 5 mg Di-tert-butyl-p-kresol wird im Vakuum eingeengt, und der Rückstand wird mit 20 ml verdünnter Salzsäure versetzt. Das ausfallende kristalline Triphenylphosphinoxid wird dreimal mit je 20 ml Essigsäureäthylester ausgewaschen. Die wäßrige Phase wird mit 20 ml Essigsäureäthylester überschichtet und mit Kaliumcarbonat alkalisch gestellt. Die organische Phase wird über Natriumsulfat getrocknet und im Vakuum von Lösungsmittel befreit. Nach erneuter Zugabe von 5 mg Di-tert-butyl-p-kresol wird der Rückstand im Kugelrohofen bei maximal 150°C/0,93 Pa destilliert. Es werden 0,24 g (71 % d.Th.) 3-Methyl-6-vinyl-2,2'-bipyridin als farblose Flüssigkeit erhalten. UV-Absorption (in Chloroform): $\lambda = 250$ nm ($\varepsilon = 15\,450$; $\lambda = 293$ nm ($\varepsilon = 7700$).

Analyse für $C_{13}H_{12}N_2$:

| | | | |
|---|---|---|---|
| berechnet | C 79,56% | H 6,16% | N 14,27% |
| gefunden | C 79,05% | H 6,27% | N 13,92%. |

Beispiel 2

4-Methyl-4'-vinyl-2,2'-bipyridin

a) Herstellung von 4,4'-Dimethyl-2,2'-bipyridin-1-oxid

27,6 g (0,15 Mol) 4,4'-Dimethyl-2,2'-bipyridin werden in 300 ml Dichlormethan gelöst. Bei 23—28°C werden 86 ml einer 1,837-molaren Lösung von Peressigsäure in Essigsäureäthylester zugetropft. Nach 50 Stunden Rühren bei 23°C wird im Vakuum eingeengt. Aus Äthylbenzol/Petroläther kristallisieren 27,2 g (90% d.Th.) 4,4'-Dimethyl-2,2'-bipyridin-1-oxid; Fp. 80—130°C (unscharf).

Analyse für $C_{12}H_{12}N_2O$:

| | | | | |
|---|---|---|---|---|
| berechnet | C 71,98% | H 6,04% | N 13,99% | O 7,99% |
| gefunden | C 71,70% | H 6,23% | N 13,97% | O 8,16%. |

b) Herstellung von 4-Methyl-4'-acetoxymethyl-2,2'-bipyridin

25,5 g (0,127 Mol) 4,4'-Dimethyl-2,2'-bipyridin-1-oxid in 130 ml Chloroform werden zu 260 ml Acetanhydrid gegeben. Nach 20 Stunden Rühren bei 60—65°C werden im Vakuum Chloroform und Acetanhydrid weitgehend abdestilliert. Der Rückstand wird mit 250 ml Wasser sowie 250 ml Essigsäureäthylester/THF (Volumenverhältnis 2 : 1) versetzt und mit Kaliumcarbonat alkalisch gestellt. Die Wasserphase wird mit 150 ml Essigsäureäthylester/THF (Volumenverhältnis 2 : 1) gewaschen. Die vereinigten organischen Phasen werden über Natriumsulfat getrocknet. Nach dem Entfernen des Lösungsmittels im Vakuum wird ein schwarzes, mit Kristallen vermischtes Öl erhalten. Durch Auswaschen mit Diäthyläther sowie Sublimieren bei ca. 100°C/0,093 Pa wird die Hauptmenge des kristallinen Anteils abgetrennt. Nach zweimaliger Destillation bei 122—123°C/0,093 Pa werden 14,3 g eines uneinheitlichen Produktes erhalten, welches direkt zur weiteren Umsetzung gelangt.
IR (NaCl): 1770 (vs), 1620 (s), 1230 (vs) $cm^{-1}$.

c) Herstellung von 4-Methyl-4'-hydroxymethyl-2,2'-bipyridin

Eine Mischung aus 9,4 g des gemäß Beispiel 3 (b) erhaltenen Rohproduktes, 130 ml 10%iger Natronlauge sowie 50 ml Äthanol wird 30 Minuten unter Rückfluß gekocht. Anschließend wird das Äthanol abdestilliert, und der Rückstand wird mit Salzsäure angesäuert. Nach dem Auswaschen mit 200 ml Essigsäureäthylester wird die Wasserphase mit Kaliumcarbonat alkalisch gestellt, und das Reaktionsgemisch wird zweimal mit je 80 ml Essigsäureäthylester extrahiert. Nach dem Trocknen über Natriumsulfat und dem Behandeln mit Aktivkohle wird im Vakuum eingeengt. Durch Zugabe von Petroläther erhält man 3,1 g Kristalle, welche aus Diäthyläther umkristallisiert werden. Ausbeute an 4-Methyl-4'-hydroxymethyl-2,2'-bipyridin: 1,6 g (5,3 % d. Th.), bezogen auf das 4,4'-Dimethyl-2,2'-bipyridin-1-oxid-Ausgangsprodukt; Fp. 110–112°C.

Analyse für $C_{12}H_{12}N_2O$:

| | | | | |
|---|---|---|---|---|
| berechnet | C 71,98% | H 6,04% | N 13,99% | O 7,99% |
| gefunden | C 72,10% | H 6,10% | N 13,85% | O 8,03%. |

Die NMR-spektroskopisch bestimmte Ausbeute nach der Aufarbeitung der Mutterlauge beläuft sich auf ca. 30 bis 40% d. Th., bezogen auf das 4,4'-Dimethyl-2,2'-bipyridin-1-oxid. Das entstandene Nebenprodukt (4,4'-Dimethyl-3-hydroxy-2,2'-bipyridin) kann durch zweimalige Sublimation bei 70–90°C/0,67 Pa und anschließendes Umkristallisieren aus Cyclohexan abgetrennt werden; Fp. 88–90°C.

$^1$H–NMR ($CDCl_3$) $\delta = 2{,}33$ (s; 3 H); 2,43 (s; 3 H); 7,08 (m; 2 H), 8,06 (m; 1 H); 8,31 (m; 1 H); 8,42 (m; 1 H); 9,57 (s; 1 H) ppm.

d) Herstellung von 4-Methyl-4'-brommethyl-2,2'-bipyridin

1,5 g 4-Methyl-4'-hydroxymethyl-2,2'-bipyridin werden analog Beispiel 2 (e) umgesetzt. Man erhält 1,74 g (88 % d. Th.) 4-Methyl-4'-brommethyl-2,2'-bipyridin als unbeständiges Öl, das unmittelbar zur weiteren Umsetzung verwendet wird.

e) Herstellung von [4-Methyl-2,2'-bipyridin-4'-yl(methylen)]triphenylphosphoniumbromid

1,74 g (6,6 mMol) 4-Methyl-4'-brommethyl-2,2'-bipyridin werden analog Beispiel 2 (f) umgesetzt. Nach dem Umkristallisieren aus Wasser werden 2,7 g (77 % d. Th.) [4-Methyl-2,2'-bipyridin-4'-yl(methylen)]triphenylphosphoniumbromid erhalten; Fp. 260–265°C.

Analyse für $C_{30}H_{26}BrN_2P \cdot 0{,}381\ H_2O$:

| | | | | | |
|---|---|---|---|---|---|
| berechnet | C 67,70% | H 5,07% | N 5,26% | Br 15,01% | P 5,82% |
| gefunden | C 67,78% | H 5,29% | N 5,27% | Br 14,91% | P 5,94%. |

f) Herstellung von 4-Methyl-4'-vinyl-2,2'-bipyridin

1,4 g (2,7 mMol) [4-Methyl-2,2'-bipyridin-4'-yl(methylen)]triphenylphosphoniumbromid werden analog Beispiel 2 (g) umgesetzt. Man erhält 0,36 g (70% d. Th.) 4-Methyl-4'-vinyl-2,2'-bipyridin in Form farbloser Kristalle; Fp. 82–86°C. UV-Absorption (in Chloroform): $\lambda_{max} = 281$ nm ($\varepsilon = 10\,600$).

Analyse für $C_{13}H_{12}N_2$:

| | | | |
|---|---|---|---|
| berechnet | C 79,56% | H 6,16% | N 14,27% |
| gefunden | C 79,45% | H 6,20% | N 14,15%. |

Beispiel 3

Bis-(2,2'-Bipyridin)-(4-vinyl-6-methyl-2,2'-bipyridin)-Ruthenium(II)perchlorat

$[Ru(bipy)_2(4V6Mebipy)](ClO_4)_2$

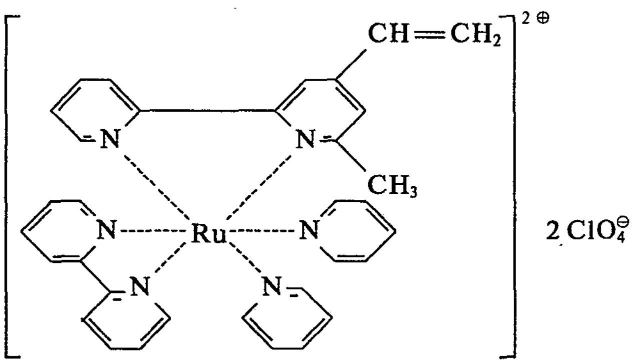

0,176 g ($8,97 \times 10^{-4}$ Mol) 4-Vinyl-6-methyl-2,2'-bipyridin werden in 150 ml entgastem Äthanol gelöst. Nach Zugabe von 0,5 g ($8,99 \times 10^{-4}$ Mol) Dichloro-bis(2,2'-bipyridin)-Ruthenium(II) mit ca. 4 Äquivalenten Kristallwasser wird 2 Stunden unter Stickstoffatmosphäre zum Rückfluß erhitzt. Das Lösungsmittel wird im Vakuum entfernt, und der Rückstand wird in ca. 100 ml Wasser gelöst. Durch Zugabe von 10 ml 1 N Perchlorsäure werden rote Kristalle ausgefällt. Nach dem Umkristallisieren aus heißem Wasser und Trocknen bei 25°C/0,13 Pa werden 0,693 g (94% d.Th.) des obigen Produkts erhalten.
UV/Vis-Absorption (in Wasser): $\lambda = 244$ ($\varepsilon = 33\,650$), 287 ($\varepsilon = 65\,500$), 452 nm ($\varepsilon = 13\,650$).

Analyse für $C_{33}H_{28}N_6Cl_2O_8Ru \cdot$ (0,68 $H_2O$:

| | | | | | | |
|---|---|---|---|---|---|---|
| berechnet | C 48,29% | H 3,62% | N 10,24% | Cl 8,64% | Ru 12,31% | $H_2O$ 1,49% |
| gefunden | C 48,35% | H 3,49% | N 10,22% | Cl 8,15% | Ru 12,4 % | $H_2O$ 1,49%. |

Beispiel 4

Dichloro-(4-vinyl-6-methyl-2,2'-bipyridin)Platin(II)

$[Pt(4V6Mebipy)Cl_2]$

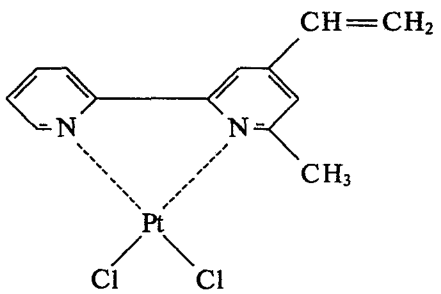

Ein Gemisch aus 0,830 g ($2 \times 10^{-3}$ Mol) Kaliumtetrachloroplatinat in 100 ml Wasser, 0,432 g ($2,2 \times 10^{-3}$ Mol) 4-Vinyl-6-methyl-2,2'-bipyridin und 4 ml 2 N Salzsäure wird 30 Minuten gekocht. Nach dem Abkühlen werden die ausgefallenen gelben Kristalle abgesaugt. Nach Eindampfen des Filtrats wird weiteres Produkt erhalten. Es wird aus N,N-Dimethylacetamid/Wasser umkristallisiert.

Analyse für $C_{13}H_{12}Cl_2N_2Pt$:

| | | | | | |
|---|---|---|---|---|---|
| berechnet | C 33,78% | H 2,62% | N 6,06% | Cl 15,34% | Pt 42,20% |
| gefunden | C 34,01% | H 2,82% | N 6,20% | Cl 15,27% | Pt 41,8 %. |

## Beispiel 5

Poly-[1-(6-methyl-2,2'-bipyridin-4-yl)-äthylen

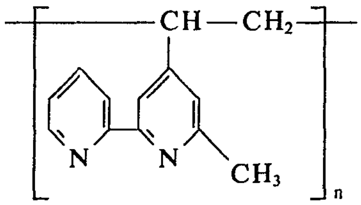

4 g (0,0204 Mol) frisch destilliertes 4-Vinyl-6-methyl-2,2'-bipyridin werden in einer mit Stickstoff gespülten Ampulle mit 10 mg Azobisisobutyronitril (AIBN) versetzt. Es wird 16 Stunden unter Luftausschluß auf 70°C erhitzt. Das entstandene glasartige Produkt wird in Chloroform gelöst. Durch Eingießen der Lösung in ca. 250 ml Diäthyläther wird ein weißes Pulver erhalten. Ausbeute 3,8 g (95 % d. Th.). Grenzviskosität (Chloroform): $[\eta]$ = 1,0 dl/g. Durchschnittliches Molekulargewicht $\overline{M}_w$ = 530 000. Glasumwandlungstemperatur Tg = 141°C. UV-Absorption (in Chloroform): $\lambda$ = 240 ($\varepsilon$ = 10 600), 288 nm ($\varepsilon$ = 12 100).

Analyse für $(C_{13}H_{12}N_2)_n$:
berechnet C 79,56 % H 6,16 % N 14,27 %
gefunden C 79,28 % H 6,20 % N 14,23 %.

## Beispiel 6

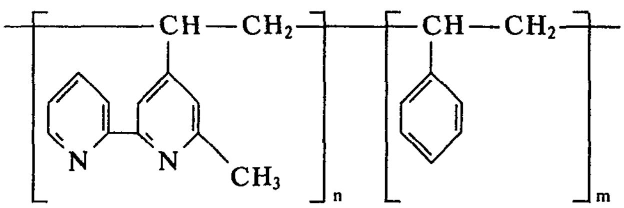

$$\frac{n}{n+m} = 0,53$$

1 g (5,1 × $10^{-3}$ Mol) frisch destilliertes 4-Vinyl-6-methyl-2,2'-bipyridin sowie 0,47 g (4,5 × $10^{-3}$ Mol) frisch destilliertes Styrol werden in einer mit Stickstoff gespülten Ampulle mit 5 mg AIBN gemischt und analog Beispiel 6 polymerisiert. Durch Fällen aus Tetrahydrofuran/n-Hexan werden 1,4 g (95 % d. Th.) weißes, faseriges Produkt erhalten. Grenzviskosität (Chloroform): $[\eta]$ = 2,5 dl/g. $\overline{M}_w$ = 1 700 000. Glasumwandlungstemperatur Tg = 138°C.
UV-Absorption (in Chloroform): $\lambda$ = 288 nm ($\varepsilon$ = 11 800).

Analyse für $(C_{13}H_{12}N_2)_n(C_8H_8)_m$ mit $\frac{n}{n+m} = 0,53$:
berechnet C 83,62 % H 6,67 % N 9,33 %
gefunden C 83,08 % H 6,61 % N 9,95 %.

## Beispiel 7

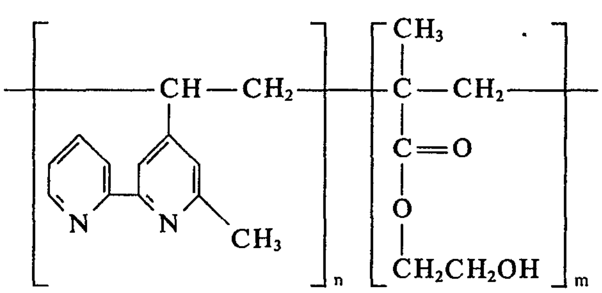

$$\frac{n}{n+m} = 0,5$$

Analog Beispiel 7 werden 1 g 4-Vinyl-6-methyl-2,2'-bipyridin und 0,664 g (5,9 × $10^{-3}$ Mol) 2-Hydroxyäthylmethacrylat copolymerisiert. Das Polymerisat löst sich unvollständig (Gelteilchen) in N,N-Dimethylformamid. Es wird aus Wasser gefällt und gefriergetrocknet.

Analyse für $(C_{13}H_{12}N_2)_n(C_6H_{10}O_3)_m$ mit $\frac{n}{n+m} = 0{,}5$:

berechnet C 69,92 % H 6,79 % N 8,58 %
gefunden C 69,48 % H 6,56 % N 8,76 %.

Beispiel 8

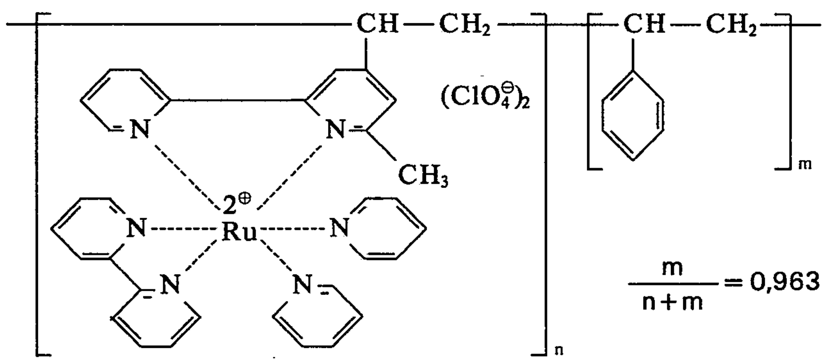

$\frac{m}{n+m} = 0{,}963$

64 mg (7,91 $\times 10^{-5}$ Mol) des gemäß Beispiel 4 hergestellten Bis-(2,2′-Bipyridin)-(4-vinyl-6-methyl-2,2′-bipyridin)-Ruthenium(II)perchlorats werden in einer mit Argon gefüllten Ampulle mit einer Mischung von 2,86 mg AIBN und 1 ml N,N-Dimethylacetamid (unter Argon destilliert) versetzt. Zu der erhaltenen roten Lösung werden 165 mg frisch destilliertes Styrol gegeben. Es wird 19 Stunden unter Luftausschluß auf 70°C erhitzt. Nach Eingießen der Lösung in 15 ml Methanol und Zentrifugieren wird ein orangefarbenes Produkt erhalten, welches durch wiederholtes Aufschlämmen in Methanol und anschließendes Zentrifugieren gereinigt wird. Ausbeute: 46,3 mg (20 % d. Th.).
UV/Vis-Absorption (in N,N-Dimethylformamid): $\lambda = 290, 444$ nm.

Analyse für $(C_{33}H_{28}N_6Cl_2O_8Ru)_n(C_8H_8)_m$ mit $\frac{m}{n+m} = 0{,}963$:
berechnet C 82,25 % H 6,75 % N 2,39 % Cl 2,20 %
gefunden C 82,31 % H 6,76 % N 2,29 % Cl 2,22 %.

Beispiel 9

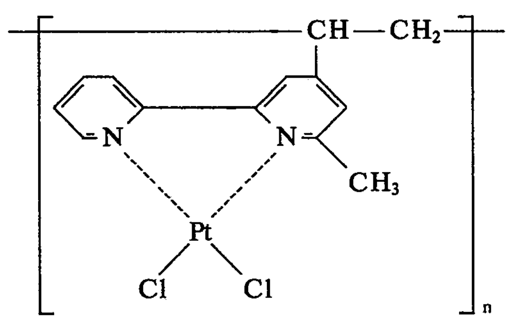

Analog Beispiel 6 werden 300 mg (6,49 $\times 10^{-4}$ Mol) des gemäß Beispiel 5 hergestellten Dichloro-(4-vinyl-6-methyl-2,2′-bipyridin)Platin(II), gelöst in 1 ml N,N-Dimethylacetamid, in Gegenwart von 1,298 $\times 10^{-5}$ Mol AIBN polymerisiert. Aus der dunkelgelben Lösung fällt ein grüngelber Niederschlag aus. Die darüberstehende flüssige Phase wird farblos. Der Niederschlag wird mit Wasser, Methanol und Diäthyläther gewaschen. Es werden 277 mg (92 % d. Th.) eines gelben unlöslichen Pulvers erhalten.

Analyse für $(C_{13}H_{12}Cl_2N_2Pt)_n$:
berechnet C 33,78 % H 2,62 % N 6,06 % Cl 15,34 % Pt 42,20 %
gefunden C 34,20 % H 2,81 % N 6,21 % Cl 15,32 % Pt 41,98 %.

## Beispiel 10

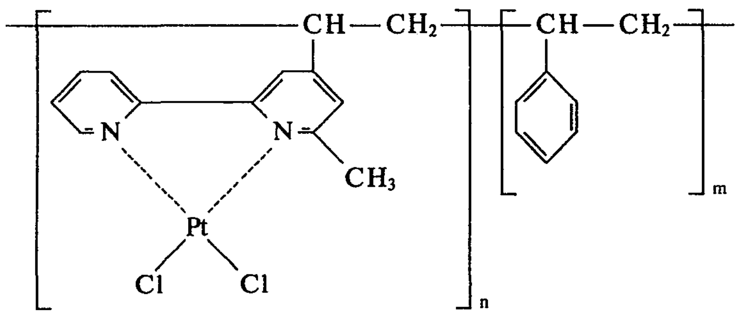

Analog Beispiel 9 werden 98,3 mg ($2{,}127 \times 10^{-4}$ Mol) des gemäß Beispiel 5 hergestellten Dichloro-(4-vinyl-6-methyl-2,2'-bipyridin)Platin(II), gelöst in 2,7 ml N,N-Dimethylacetamid, und 443 mg ($4{,}253 \times 10^{-4}$ Mol) Styrol in Gegenwart von $4{,}466 \times 10^{-5}$ Mol AIBN copolymerisiert. Nach dem Ausfällen aus 125 ml Diäthyläther, Waschen des Niederschlages mit Diäthyläther und Methanol werden 116,4 mg (22 % d. Th.) hellgelbes Produkt erhalten. $\eta_{red}$ (0,5 % in N,N-Dimethylacetamid) = 0,11 dl/g.

## Beispiel 11

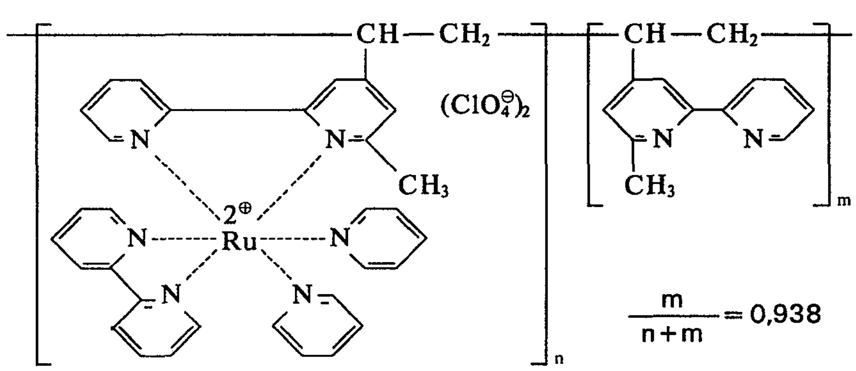

$$\frac{m}{n+m} = 0{,}938$$

0,176 g ($8{,}97 \times 10^{-4}$ Mol Wiederholungseinheiten) des gemäß Beispiel 6 hergestellten Poly-[1-(6-methyl-2,2'-bipyridin-4-yl)-äthylens] werden in 150 ml entgastem N,N-Dimethylformamid gelöst. Nach Zugabe von 1 g ($1{,}80 \times 10^{-3}$ Mol) Dichloro-bis-(2,2'-bipyridin)Ruthenium(II) mit ca. 4 Äquivalenten Kristallwasser wird 15 Stunden unter Stickstoffatmosphäre auf 140°C erhitzt. Das Lösungsmittel wird im Vakuum weitgehend entfernt, und der Rückstand wird in eine Mischung aus 10 ml 1 N Perchlorsäure und 100 ml Wasser gegossen. Der Niederschlag wird abzentrifugiert und mehrmals mit Wasser sowie Methanol gewaschen. Es werden 165 mg eines ziegelroten Pulvers erhalten. Aus der UV/Vis-Absorption (in N,N-Dimethylformamid) bei $\lambda = 452$ nm ($\varepsilon = 42$ bei $c = 2 \times 10^{-2}$ g/100 ml) errechnet sich die Copolymerzusammensetzung $\frac{m}{m+n} = 0{,}938$.

## Beispiel 12

Umsetzungsprodukt von Poly[1-(6-methyl-2,2'-bipyridin-4-yl)äthylen] mit Tetraacetonitrilo-Kupfer(I)perchlorat. Zu einer Lösung von 1,5 g ($4{,}33 \times 10^{-3}$ Mol) Tetraacetonitrilo-Kupfer(I)perchlorat in 50 ml N,N-Dimethylformamid wird eine Lösung von 0,85 g ($4{,}33 \times 10^{-3}$ Mol Wiederholungseinheiten) des gemäß Beispiel 6 hergestellten Poly[1-(6-methyl-2,2'-bipyridin-4-yl)äthylens] in 25 ml N,N-Dimethylformamid gegeben. Das Reaktionsgemisch wird 15 Stunden bei 23°C gerührt, und das entstandene rotbraune Öl wird abgesaugt. Es wird mit 350 ml N,N-Dimethylformamid und anschließend mit 500 ml Wasser sowie Äthanol und Diäthyläther gewaschen. Nach dem Trocknen bei 80°C/13 000 Pa werden 1,07 g (89 % d. Th.) feinpulveriges Produkt erhalten.

Analyse für $(C_{26}H_{24}N_4ClO_4Cu)_n$:

| | | | | | |
|---|---|---|---|---|---|
| berechnet | C 56,22 % | H 4,35 % | N 10,09 % | Cl 6,38 % | Cu 11,44 % |
| gefunden | C 51,67 % | H 4,35 % | N 9,34 % | Cl 6,16 % | Cu 12,4 %. |

Beispiel 13

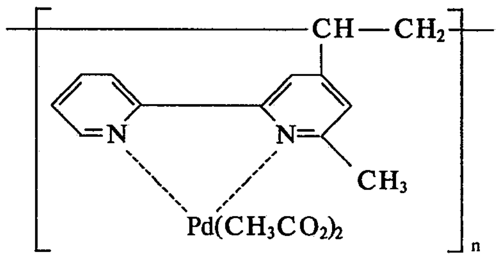

Zu einer Lösung von 0,595 g ($3{,}03 \times 10^{-3}$ Mol) Poly[1-(6-methyl-2,2'-bipyridin-4-yl)äthylen] in 90 ml Tetrahydrofuran wird eine Lösung von 0,697 g Palladium(II)acetat in 15 ml Tetrahydrofuran gegeben. Es wird 16 Stunden bei 23°C gerührt. Der gelbbraune Niederschlag wird abgesaugt und mit ca. 250 ml Tetrahydrofuran gewaschen. Ausbeute 1,08 g (85% d. Th.).

Analyse für $(C_{17}H_{18}N_2O_4Pd)_n$:
berechnet C 48,53% H 4,31% N 6,66% Pd 25,29%
gefunden C 47,89% H 4,42% N 6,65% Pd 24,4%.

Beispiel 14

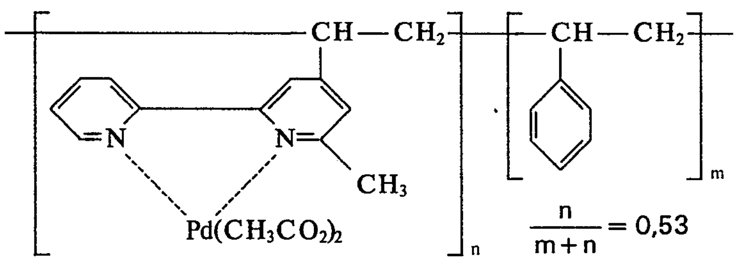

$\frac{n}{m+n} = 0{,}53$

Analog Beispiel 14 werden 300,4 mg (1 mMol) des gemäß Beispiel 7 hergestellten Copolymeren mit 230 mg Palladium(II)acetat umgesetzt. Ausbeute 420 mg (80% d. Th.).

Analyse für $(C_{17}H_{18}N_2O_4Pd)_n(C_8H_8)_m$ mit $\frac{n}{n+m} = 0{,}53$:
berechnet C 56,40% H 4,93% N 5,46% Pd 20,74%
gefunden C 56,36% H 5,30% N 5,34% Pd 19,70%.

Beispiel 15

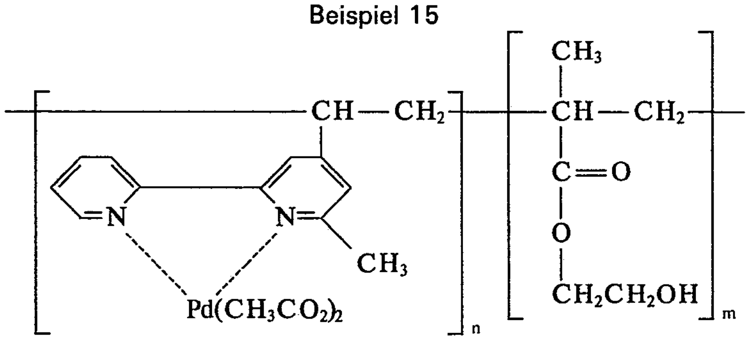

$\frac{n}{n+m} = 0{,}5$

Analog Beispiel 14 werden 326,4 mg (1 mMol) des gemäß Beispiel 8 erhaltenen Copolymeren mit 320 mg Palladium(II)acetat umgesetzt. Ausbeute 436 mg (79 d. Th.).

Analyse für $(C_{17}H_{18}N_2O_4Pd)_n(C_6H_{10}O_3)_m$ mit $\frac{n}{n+m} = 0{,}5$:
berechnet C 50,15% H 5,12% N 5,09% Pd 19,31%
gefunden C 50,24% H 5,29% N 5,24% Pd 18,50%.

Beispiel 16

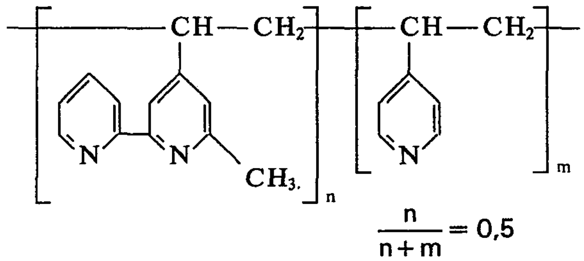

$$\frac{n}{n+m} = 0,5$$

Eine Mischung aus 2 g ($1,02 \times 10^{-2}$ Mol) frisch destilliertem 4-Vinyl-6-methyl-2,2'-bipyridin und 1,17 g ($1,02 \times 10^{-2}$ Mol) frisch destilliertem 4-Vinylpyridin wird in 10 ml N,N-Dimethylacetamid gelöst. Die Lösung wird mit einer mit Stickstoff gespülten Ampulle mit 10 mg ($6,083 \times 10^{-5}$ Mol) AIBN versetzt und analog Beispiel 6 polymerisiert. Durch Eingießen der Lösung in Wasser wird ein weißes Pulver erhalten. Das Produkt wird in Tetrahydrofuran (THF) gelöst und aus Diäthyläther umgefällt. Ausbeute 2,27 g; Viskosität (Chloroform): $\eta_{red} = 0,52$ dl/g; Glasumwandlungstemperatur: Tg= 155°C.

Analyse für $(C_{13}H_{12}N_2)_n(C_7H_7N)_m$ mit $\frac{n}{n+m} = 0,50$:

berechnet C 76,63% H 6,54% N 13,41%
gefunden C 76,83% H 6,21% N 13,40%.

Beispiel 17

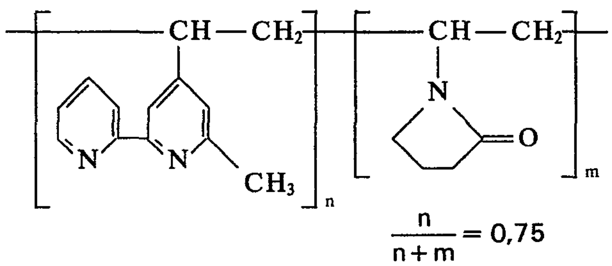

$$\frac{n}{n+m} = 0,75$$

2 g 4-Vinyl-6-methyl-2,2'-bipyridin und 1,13 g ($1,02 \times 10^{-3}$ Mol) N-Vinylpyrrolidon werden analog Beispiel 17 copolymerisiert. Das Produkt wird mit Diäthyläther gefällt und in THF gelöst. Durch Eingießen der Lösung in Wasser wird ein weißes Pulver erhalten, welches aus THF/Diäthyläther umgefällt wird. Ausbeute: 1,87 g; Viskosität (Chloroform): $\eta_{red} = 0,44$ dl/g. Glasumwandlungstemperatur: Tg = 150°C.

Analyse für $(C_{13}H_{12}N_2)_n(C_6H_9NO)_m$ mit $\frac{n}{n+m} = 0,75$:

berechnet C 77,22% H 6,48% N 14,01% O 2,29%
gefunden C 77,47% H 6,33% N 13,82% O 2,26%.

Beispiel 18

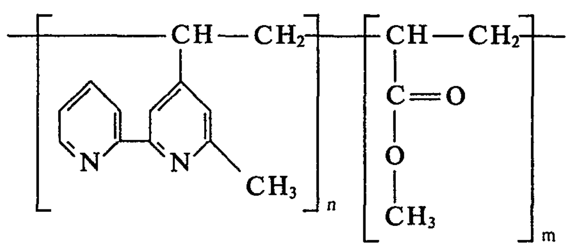

$$\frac{n}{n+m} = 0,66$$

2 g 4-Vinyl-6-methyl-2,2'-bipyridin und 0,87 g ($1,02 \times 10^{-2}$ Mol) Methylacrylat werden analog Beispiel 17 copolymerisiert. Das Produkt wird mit Wasser gefällt und aus THF/Cyclohexan umgefällt. Es

wird ein weißes Pulver erhalten. Ausbeute: 2,18 g; Viskosität (Chloroform): $\eta_{red} = 0{,}36$ dl/g; Glasumwandlungstemperatur: Tg = 120°C.

Analyse für $(C_{13}H_{12}N_2)_n(C_4H_6O_2)_m$ mit $\frac{n}{n+m} = 0{,}66$:

berechnet C 74,86% H 6,33% N 11,45% O 7,36%
gefunden C 74,22% H 6,26% N 11,60% O 7,73%.

Beispiel 19

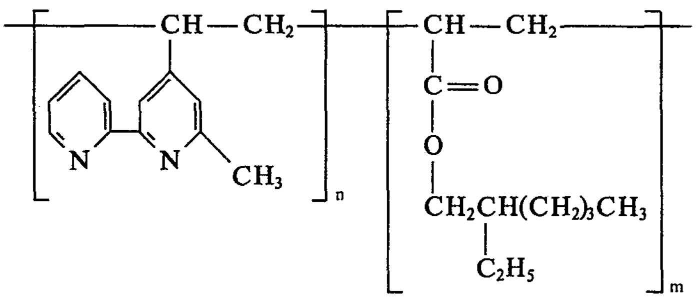

$$\frac{n}{n+m} = 0{,}63$$

2 g 4-Vinyl-6-methyl-2,2'-bipyridin und 1,87 g ($1{,}02 \times 10^{-2}$ Mol) 2-Äthylhexylacrylat werden analog Beispiel 17 copolymerisiert. Das Produkt wird durch Eingießen in eine Mischung aus 400 ml Wasser, 300 ml Isopropanol und 100 ml Methanol bei –10° bis –15°C als flockiges Produkt erhalten, welches bei Raumtemperatur zu einem hochviskosen Öl zerfließt. Dieses wird in THF gelöst und aus Methanol bei –25°C umgefällt. Nach dem Trocknen wird ein farbloses Pulver erhalten. Ausbeute: 2,46 g; Viskosität (Chloroform): $\eta_{red} = 0{,}34$ dl/g; Glasumwandlungstemperatur: Tg = 72°C.

Analyse für $(C_{13}H_{12}N_2)_n(C_{11}H_{20}O_2)_m$ mit $\frac{n}{n+m} = 0{,}63$:

berechnet C 76,77% H 7,86% N 9,20% O 6,17%
gefunden C 76,80% H 7,67% N 9,54% O 6,37%.

Beispiel 20

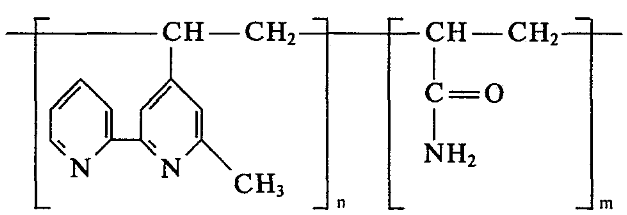

$$\frac{n}{n+m} = 0{,}67$$

2 g 4-Vinyl-6-methyl-2,2'-bipyridin und 0,72 g ($1{,}02 \times 10^{-2}$ Mol) Acrylamid werden analog Beispiel 17 copolymerisiert. Das Produkt wird aus Wasser gefällt und in THF gelöst. Die Lösung wird filtriert, und das klare Filtrat wird in Diäthyläther eingerührt. Es wird ein weißes Pulver erhalten. Ausbeute: 1,88 g; Viskosität (Chloroform): $\eta_{red} = 0{,}39$ dl/g; Glasumwandlungstemperatur: Tg = 156°C.

Analyse für $(C_{13}H_{12}N_2)_n(C_3H_5NO)_m$ mit $\frac{n}{n+m} = 0{,}67$:

berechnet C 75,19% H 6,30% N 15,10% O 3,41%
gefunden C 75,61% H 6,24% N 14,57% O 3,79%.

Beispiel 21

Polymeres aus n Mol 6-Methyl-4-vinyl-2,2'-bipyridin, m Mol Styrol und o Mol Divinylbenzol mit $\frac{n}{n+m+o} = 0{,}1$.

Zu 40 ml einer 0,1prozentigen Lösung von Polyvinylalkohol in Wasser (Serum) wird eine Mischung aus 3 g ($1{,}53 \times 10^{-2}$ Mol) frisch destilliertem 4-Vinyl-6-methyl-2,2'-bipyridin, 14,08 g (0,135 Mol) Styrol, 400 mg ($3{,}06 \times 10^{-3}$ Mol, 2 Mol.-%) technischem Divinylbenzol sowie 75,6 mg

($4{,}6 \times 10^{-4}$ Mol) AIBN gegeben. Die Suspension wird 14 Stunden bei 70°C und 6 Stunden bei 100°C gerührt (700 UpM). Das weiße feinkörnige Produkt wird abgesaugt, mit Wasser gewaschen und 2 Stunden in 200 ml THF in die Siedehitze gequollen. Das Gel wird in 4 Liter Wasser eingerührt und nach dem Abtrennen 20 Stunden in einem Soxhlet-Extraktor mit THF extrahiert. Das Gel wird 16 Stunden bei 80°C/2666 Pa, anschließend 16 Stunden bei 60°C/1,33 Pa getrocknet. Ausbeute: 15,2 g; Quellfaktor (THF): Q = 2,4.

Analyse für $(C_{13}H_{12}N_2)_n(C_8H_8)_m(C_{10}H_{10})_o$ mit $\frac{n}{n+m+o} = 0{,}10$, $\frac{o}{n+m+o} = 0{,}02$:

berechnet C 90,07 % H 7,47 % N 2,46 %
gefunden C 88,68 % H 7,37 % N 2,57 %.

Beispiel 22

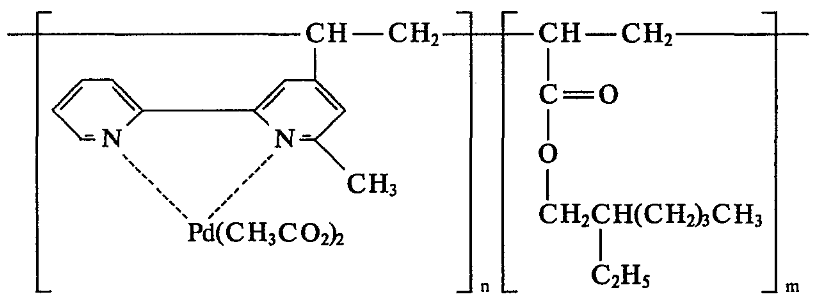

$\frac{n}{n+m} = 0{,}63$

Analog Beispiel 13 werden 500 mg (2,61 mMol Bipyridin-Einheiten) des gemäß Beispiel 19 erhaltenen Copolymeren mit 644 mg (2,87 mMol) Palladium(II)acetat umgesetzt. Es werden nach Extraktion mit THF und Trocknen 850 mg eines hellbraunen Pulvers erhalten. Das Produkt hat einen Palladiumgehalt von 24,7 Gew.%.

Beispiel 23

Polymeres aus n Mol 6-Methyl-4-vinyl-2,2'-bipyridin, m Mol Styrol und o Mol Divinylbenzol mit $\frac{n}{n+m+o} = 0{,}1$, voll komplexiert mit Palladium(II)acetat.

Analog Beispiel 13 werden 2,7 g des gemäß Beispiel 21 erhaltenen Copolymeren mit 670 mg Palladium(II)acetat umgesetzt, wobei das Copolymere als gequollenes Gel suspendiert wird. Es werden nach Extraktion mit THF und Trocknen 3,06 g eines braunen Produktes mit einem Palladiumgehalt von 8,48 Gew.% erhalten. Quellungsgrad (THF): Q = 2,2.

Beispiel b

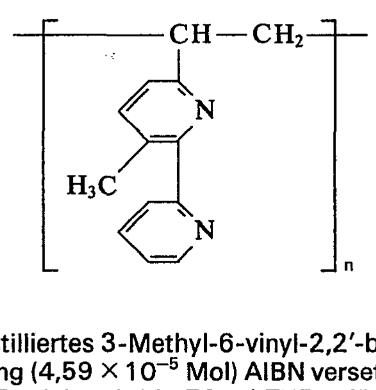

3 g ($1{,}53 \times 10^{-2}$ Mol) frisch destilliertes 3-Methyl-6-vinyl-2,2'-bipyridin werden in einer mit Stickstoff gespülten Ampulle mit 7,54 mg ($4{,}59 \times 10^{-5}$ Mol) AIBN versetzt und analog Beispiel 5 polymerisiert. Das entstandene glasartige Produkt wird in 70 ml THF gelöst. Durch Eingießen der Lösung in 1000 ml n-Hexan wird ein weißes Pulver erhalten. Ausbeute: 2,85 g; Grenzviskosität (Chloroform): $[\eta] = 1{,}27$ dl/g; durchschnittliches Molekulargewicht: $\overline{M}_w = 520\,000$; Glasumwandlungstemperatur Tg = 107°C.

Analyse für $(C_{13}H_{12}N_2)_n$:
berechnet C 79,56 % H 6,16 % N 14,27 %
gefunden C 78,12 % H 5,89 % N 14,23 %.

Beispiel c

Polymeres aus n Mol 3-Methyl-6-vinyl-2,2'-bipyridin, m Mol Styrol und o Mol Divinylbenzol mit $\frac{n}{n+m+o} = 0{,}1$.

1,5 g ($7{,}64 \times 10^{-3}$ Mol) 3-Methyl-6-vinyl-2,2'-bipyridin, 7,04 g ($6{,}73 \times 10^{-2}$ Mol) Styrol und 200 mg ($1{,}53 \times 10^{-3}$ Mol, 2 Mol.%) technisches Divinylbenzol werden mittels 37,8 mg ($2{,}3 \times 10^{-4}$ Mol) AIBN gemäß Beispiel 21 in 20 ml Serum copolymerisiert und aufgearbeitet. Ausbeute: 7,2 g; Quellfaktor (THF): Q = 2,5.

Analyse für $(C_{13}H_{12}N_2)_n(C_8H_8)_m(C_{10}H_{10})_o$ mit $\frac{n}{n+m+o} = 0{,}1$ und $\frac{o}{n+m+o} = 0{,}02$:

berechnet C 90,07 % H 7,47 % N 2,46 %
gefunden C 89,55 % H 7,86 % N 2,51 %.

Beispiel d

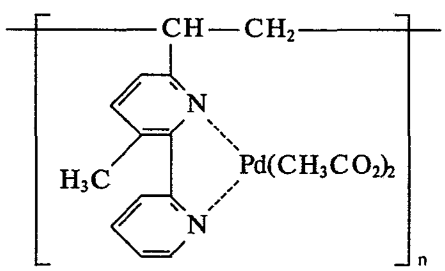

Analog Beispiel 13 werden 200 mg Poly[1-(3-methyl-2,2'-bipyridin-6-yl)äthylen] mit 275 mg Palladium(II)acetat umgesetzt. Es werden 430 mg eines hellbraunen Pulvers mit einem Palladiumgehalt von 26,2 Gew.% erhalten.

Beispiel e

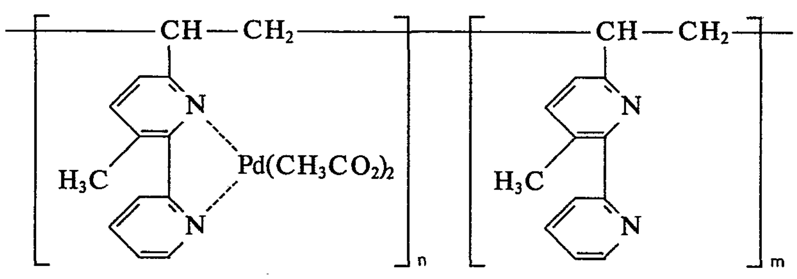

$\frac{n}{m+n} = 0{,}1$

Analog Beispiel 13 werden 300 mg Poly[1-(3-methyl-2,2'-bipyridin-6-yl)äthylen] mit 34,3 mg Palladium(II)acetat umgesetzt. Es werden 330 mg eines beigen Pulvers mit einem Palladiumgehalt von 4,58 Gew.% erhalten.

Beispiel f

Polymeres aus n Mol 3-Methyl-6-vinyl-2,2'-bipyridin, m Mol Styrol und o Mol Divinylbenzol mit $\frac{n}{n+m+o} = 0{,}1$, voll komplexiert mit Palladium(II)acetat.

Analog Beispiel 23 wird das gemäß Beispiel c erhaltene Copolymere mit Palladium(II)acetat umgesetzt. Ausbeute: 3,0 g. Quellungsgrad (THF): Q = 2,1, Palladiumgehalt: 8,5 Gew.%.

## Anwendungsbeispiele

## Verwendung als Umvinylierungskatalysator

### Beispiel I

0,225 g des nach Beispiel 13 hergestellten Polymeren (Katalysator) mit einem Palladiumgehalt von 24,4 Gew.% werden zusammen mit 12,2 g (0,1 Mol) Benzoesäure und 0,005 g 2,6-Di-tert-butyl-p-kresol in 55,4 ml Essigsäurevinylester suspendiert. Die Reaktionsmischung wird unter trockenem Stickstoff auf 60—65°C erwärmt, wobei der Reaktionsablauf durch Gaschromatographie verfolgt wird. Nach insgesamt 22 Stunden wird vom Katalysator abdekantiert, von entstandener Essigsäure und nichtumgesetzter Benzoesäure durch Ausschütteln mit wäßriger Natriumbicarbonat-Lösung befreit, und der Benzoesäurevinylester wird durch fraktionierte Destillation isoliert. Ausbeute an Benzoesäurevinylester = 11 g (0,074 Mol, entsprechend 74 % d. Th.); Kp. 94°/1064 Pa. Nach Abtrennen des Katalysators hat das Reaktionsgemisch einen Pd-Gehalt unterhalb 2 ppm.

Der abdekantierte Katalysator wird erneut in der Mischung aus Benzoesäure, 2,6-Di-tert-butyl-p-kresol und Vinylacetat suspendiert und unter Stickstoff auf 60—65°C erwärmt. Nach weiteren 31 Stunden sind wiederum 10,6 g = 0,072 Mol Benzoesäurevinylester entstanden, entsprechend einer Ausbeute von 72 % d. Th.

### Beispiel II

Analog der in Beispiel I beschriebenen Arbeitsweise werden 0,285 g des nach Beispiel 15 hergestellten Polymeren mit einem Palladiumgehalt von 18,5 Gew.% mit 12,2 g Benzoesäure und 55,4 ml Vinylacetat auf 60—65°C erwärmt. Nach 29 Stunden sind 11,3 g Benzoesäurevinylester entstanden, entsprechend einer Ausbeute von 76 % d. Th. Mit dem wiedergewonnenen Katalysator werden in einem weiteren Reaktionsansatz nach 20 Stunden erneut 10,4 g Benzoesäurevinylester erhalten, entsprechend 70,5 % d. Th.

### Beispiel III

Analog der in Beispiel I beschriebenen Arbeitsweise werden 0,28 g des nach Beispiel 14 hergestellten Polymeren mit einem Palladiumgehalt von 19,7 Gew.% mit 12,2 g Benzoesäure und 55,4 ml Vinylacetat erwärmt. Im ersten Reaktionslauf werden nach 22 Stunden 76 % d. Th. Benzoesäurevinylester isoliert. In einem weiteren Reaktionsansatz werden mit dem gleichen Polymeren nach 20 Stunden 76 % d. Th. Benzoesäurevinylester erhalten.

### Beispiel IV

Eine Mischung aus 44,4 ml (0,48 Mol) Essigsäurevinylester und 9,77 g (0,08 Mol) Benzoesäure wird auf 65°C erwärmt. Es werden 500 mg ($4 \times 10^{-4}$ Äquivalente Pd) des gemäß Beispiel 23 erhaltenen Polymeren (Katalysator) zugegeben. Nach 1, 2, 3, 5, 7, 12, 17, 24 und 30 Stunden werden jeweils 0,6 ml Reaktionsmischung entnommen und der Umsatz gaschromatographisch bestimmt (1-Chlornaphthalin als interner Standard). Die Zeit/Umsatzkurve zeigt nach ca. 15 Stunden die maximale Ausbeute an Benzolsäurevinylester von 61 % (Gleichgewichtseinstellung). Nach Abtrennen des Katalysators hat das Reaktionsgemisch einen Pd-Gehalt unter 2 ppm.

### Beispiel V

Entsprechend der in Anwendungsbeispiel IV beschriebenen Arbeitsweise werden 500 mg ($4 \times 10^{-4}$ Äquivalente Pd) des gemäß Beispiel 22 erhaltenen Polymeren als Katalysator eingesetzt. Nach 30 Stunden wird eine Ausbeute an Benzoesäurevinylester von 58,6 % gemessen.

### Vergleichsbeispiele VI—VIII

Nach der in Beispiel IV beschriebenen Methode werden zum Vergleich andere Polymere als Umvinylierungs-Katalysatoren eingesetzt. Die Ergebnisse sind in der folgenden Tabelle angegeben.

Tabelle

| Vergleichsbeispiel Nr. | Katalysator nach | Menge Katalysator (mg) | Äquivalente Pd | Ausbeute an Benzoesäurevinylester Ausbeute nach 3 Std. | 30 Std. |
|---|---|---|---|---|---|
| VI | Beispiel d | 168 | $4 \times 10^{-4}$ | 17,4% | 21,7% |
| VII | Beispiel e | 168 | $7{,}2 \times 10^{-5}$ | $\leqslant 1$% | $< 1$% |
| VIII | Beispiel f | 506 | $4 \times 10^{-4}$ | 18,1% | 24,3% |

**Patentansprüche**

1. Verbindungen der Formel I

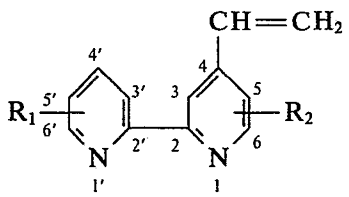

(I)

und deren Komplexe mit Metallen oder Metallverbindungen, die keine Alkalimetalle oder Erdalkalimetalle bzw. keine Alkalimetall- oder Erdalkalimetallverbindungen sind, worin $R_1$ Wasserstoff, $C_{1-6}$-Alkyl, $C_{1-6}$-Alkoxy, Phenyl oder Phenoxy und $R_2$ Wasserstoff oder Methyl bedeuten.

2. Verbindungen der Formel I und deren Komplexe nach Anspruch 1, worin $R_1$ Wasserstoff oder in 4'-, 5'- oder 6'-Stellung gebundenes $C_{1-4}$-Alkyl und $R_2$ Wasserstoff oder Methyl darstellen.

3. Verbindungen der Formel I und deren Komplexe nach Anspruch 1, worin $R_1$ in 4'- oder 6'-Stellung gebundenes Methyl und $R_2$ Wasserstoff oder $R_1$ Wasserstoff und $R_2$ in 6-Stellung gebundenes Methyl darstellen.

4. Komplexe der Verbindungen der Formel I nach Anspruch 1, worin das Metall der Nebengruppen I b oder VIII, besonders Eisen, Ruthenium, Kobalt, Nickel, Palladium, Platin oder Kupfer ist.

5. Komplexe von Verbindungen der Formel I nach Anspruch 1, worin das Metall Ruthenium, Palladium, Platin oder Kupfer ist.

6. Verfahren zur Herstellung von Verbindungen der Formel I oder Komplexen davon nach Anspruch 1, dadurch gekennzeichnet, daß man eine Verbindung der Formel II

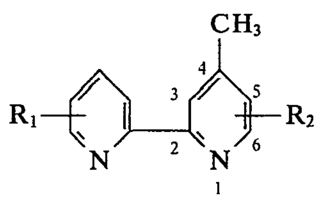

(II)

zu einer Verbindung der Formel IIIa oder IIIb

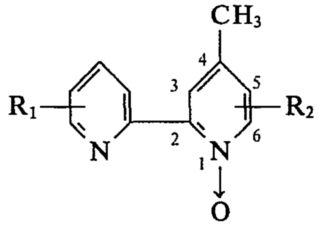

oder

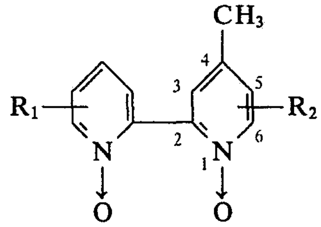

(IIIa) (IIIb)

oxidiert, die Verbindung der Formel IIIa oder IIIb mit einer Verbindung der Formel IV

$(R'CO)_2O$ (IV)

zu einer Verbindung der Formel Va oder Vb

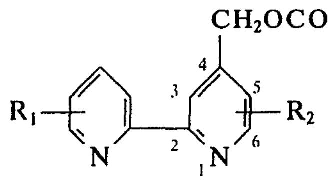

oder

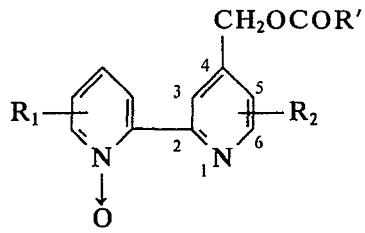

(Va) (Vb)

umsetzt, die Verbindung der Formel Vb in eine Verbindung der Formel Va überführt, die Verbindung der Formel Va in Gegenwart einer Base zu einer Verbindung der Formel VI

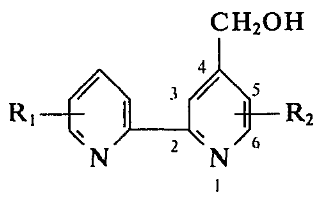

(VI)

verseift, die Verbindung der Formel VI durch Behandlung mit HCl oder HBr in die entsprechende Chlor- oder Brommethylverbindung der Formel VII

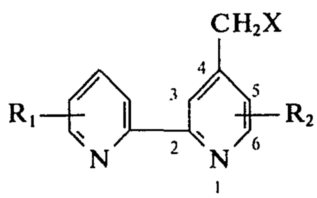

(VII)

überführt, die Verbindung der Formel VII mit einer Verbindung der Formel VIII

$P(R'')_3$ (VIII)

zu einem Phosphoniumsalz der Formel IX

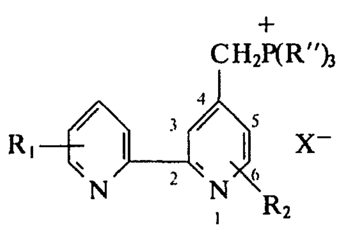

(IX)

umsetzt, das Phosphoniumsalz der Formel IX in Gegenwart einer Base mit Formaldehyd zu einer Verbindung der Formel I umsetzt und so erhaltene Verbindungen der Formel I gegebenenfalls in Komplexe mit Metallen oder Metallverbindungen, die keine Alkalimetalle oder Erdalkalimetalle bzw. keine Alkalimetall- oder Erdalkalimetallverbindungen sind, überführt, wobei $R_1$ und $R_2$ die unter Formel I angegebene Bedeutung haben, X Chlor oder Brom, R' $C_{1-5}$-Alkyl und die R'' unabhängig voneinander $C_{1-5}$-Alkyl oder unsubstituiertes oder durch $C_{1-4}$-Alkyl oder $C_{1-4}$-Alkoxy mono- oder disubstituiertes Phenyl bedeuten.

7. Verfahren zur Herstellung von Verbindungen der Formel I oder Komplexen davon nach Anspruch 1, dadurch gekennzeichnet, daß man entweder

a) eine Verbindung der Formel II

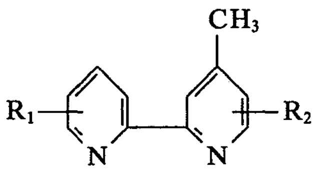

(II)

vorerst mit einem Metallierungsreagens behandelt und dann mit Formaldehyd in eine Verbindung der Formel X

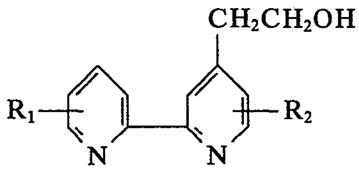

(X)

überführt,

b) eine Verbindung der Formel II vorerst mit einem Metallierungsreagens behandelt und dann mit einer Verbindung der Formel XI

$HalCH_2OCH_2R$ (XI)

zu einer Verbindung der Formel XII

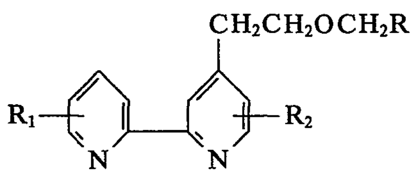

(XII)

umsetzt, die Verbindung der Formel XII zu einer Verbindung der Formel X hydriert und die Verbindung der Formel X zu einer Verbindung der Formel Ia dehydratisiert oder

c) eine Verbindung der Formel II mit einem Metallierungsreagens und einer Verbindung der Formel XI behandelt und das erhaltene Reaktionsgemisch erhitzt und so erhaltene Verbindungen der Formel I gegebenenfalls in Komplexe mit Metallen oder Metallverbindungen, die keine Alkalimetalle oder Erdalkalimetalle bzw. Alkalimetall- oder Erdalkalimetallverbindungen sind, überführt, wobei $R_1$ und $R_2$ die im Anspruch 1 angegebene Bedeutung haben, Hal ein Halogenatom und R unsubstituiertes oder durch $C_{1-6}$-Alkyl oder $C_{1-6}$-Alkoxy substituiertes Phenyl darstellen.

8. Gegebenenfalls vernetzte Polymere, die dadurch erhältlich sind, daß man 2 bis 100 Mol% einer Verbindung der Formel I und/oder eines Komplexes einer Verbindung der Formel I mit Metallen oder Metallverbindungen, die keine Alkalimetalle oder Erdalkalimetalle bzw. keine Alkalimetall- oder Erdalkalimetallverbindungen sind, gemäß Anspruch 1, und 0 bis 98 Mol.% einer Verbindung der Formel A)

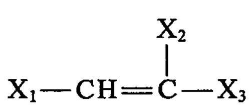

(A)

worin $X_1$ Wasserstoff, $X_2$ Wasserstoff, Chlor oder Methyl und $X_3$ Wasserstoff, Methyl, Chlor, —CN, —COOH, —$CONH_2$, Phenyl, Methylphenyl, Methoxyphenyl, Cyclohexyl, Pyridyl, Imidazolyl, Pyrrolidyl, —COO-Alkyl mit 1—12 C-Atomen im Alkyl, —COO-Phenyl,

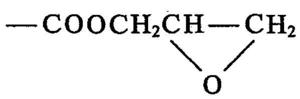

—COO-Alkyl-OH mit 1—4 C-Atomen im Alkyl, —OCO-Alkyl mit 1—4 C-Atomen im Alkyl, —OCO-Phenyl, —CO-Alkyl mit 1—3 C-Atomen im Alkyl, Alkoxy mit 1—20 C-Atomen oder Phenoxy oder $X_2$ Wasserstoff und $X_1$ und $X_3$ zusammen eine Anhydrid-Gruppierung, eine Gruppierung —CO—NR'''—CO— oder je —COOH oder —COOAlkyl mit 1—6 C-Atomen im Alkyl darstellen, wobei R''' geradkettiges oder verzweigtes $C_{1-18}$-Alkyl, Cyclohexyl oder Phenyl bedeutet, das durch $C_{1-6}$-Alkyl, Halogen, Cyano, Nitro und/oder $C_{1-3}$-Alkoxy mono- oder disubstituiert sein kann, in Gegenwart von 0 bis 60 Mol.% eines mehrfach ungesättigten Vernetzungsmittels polymerisiert und gegebenenfalls so erhaltene komplexbildende Polymere in Polymere überführt, die ganz oder teilweise mit Metallen oder Metallverbindungen komplexiert sind, die keine Alkalimetalle oder Erdalkalimetalle bzw. keine Alkalimetall- oder Erdalkalimetallverbindungen sind.

9. Gegebenenfalls vernetzte Polymere und deren Komplexe nach Anspruch 8, die dadurch erhältlich sind, daß man eine Verbindung der Formel A) verwendet, worin $X_1$ Wasserstoff, $X_2$ Wasserstoff oder Methyl und $X_3$ $-CONH_2$, Phenyl, Pyridyl, Pyrrolidyl, —COO—Alkyl—OH mit 2 bis 4 C-Atomen im Alkyl oder —COO—Alkyl mit 1 bis 12 C-Atomen im Alkyl bedeuten.

10. Vernetzte Polymere und deren Komplexe nach Anspruch 8, die dadurch erhältlich sind, daß man die Polymerisation in Gegenwart von 1 bis 30 Mol-% eines mehrfach ungesättigten Vernetzungsnittels, besonders Divinylbenzol oder Divinylpyridin, durchführt.

11. Lineare Polymere mit einem durchschnittlichen Molekulargewicht von 1000 bis 5 000 000, die aus 3 bis 100 Mol-% wiederkehrenden Strukturelementen der Formel B)

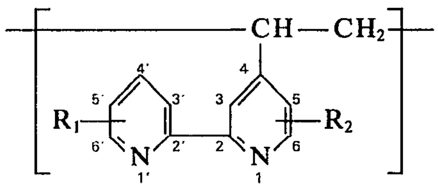

(B)

und/oder Komplexen derartiger Strukturelemente mit Metallen oder Metallverbindungen, die keine Alkalimetalle oder Erdalkalimetalle bzw. keine Alkalimetall- oder Erdalkalimetallverbindungen sind, und 0 bis 97 Mol-% wiederkehrenden Strukturelementen der Formel C)

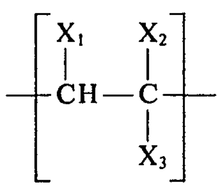

(C)

bestehen, worin $R_1$ Wassersgtoff, $C_{1-6}$-Alkyl, $C_{1-6}$-Alkoxy, Phenyl oder Phenoxy und $R_2$ Wasserstoff oder Methyl bedeuten, mit der Maßgabe, daß eines von $R_1$ und $R_2$ ungleich Wasserstoff ist, wenn die Gruppe $-CH_2-CH_2-$ in 6-Stellung gebunden ist, $X_1$ Wasserstoff, $X_2$ Wasserstoff, Chlor oder Methyl und $X_3$ Wasserstoff, Methyl, Chlor, —CN, —COOH, $-CONH_2$, Phenyl, Methylphenyl, Methoxyphenyl, Cyclohexyl, Pyridyl, Imidazolyl, Pyrrolidyl, —COO—Alkyl mit 1 bis 12 C-Atomen im Alkyl, —COO—Phenyl,

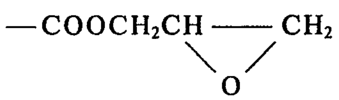

—COO—Alkyl—OH mit 1 bis 4 C-Atomen im Alkyl, —OCO—Alkyl mit 1 bis 4 C-Atomen im Alkyl, —OCO—Phenyl, —CO—Alkyl mit 1 bis 3 C-Atomen im Alkyl, Alkoxy mit 1 bis 20 C-Atomen oder Phenoxy oder $X_2$ Wasserstoff und $X_1$ und $X_3$ zusammen eine Anhyridgruppierung, eine Gruppierung —CO—NR'''—CO— oder je —COOH oder —COOAlkyl mit 1 bis 6 C-Atomen im Alkyl bedeuten, wobei R''' geradkettiges oder verzweigtes $C_{1-18}$-Alkyl, Cyclohexyl oder Phenyl darstellt, das durch $C_{1-6}$-Alkyl, Halogen, Cyano, Nitro und/oder $C_{1-3}$-Alkoxy mono- oder disubstituiert sein kann.

12. Lineare Polymere mit einem durchschnittlichen Molekulargewicht von 2000 bis 3 000 000 und/oder deren Komplexe nach Anspruch 11, die aus 3 bis 10 Mol-%wiederkehrenden Strukturelementen der Formel B) und/oder definitionsgemäßen Komplexen derartiger Strukturelemente der Formel B) und aus 0 bis 97 Mol-%wiederkehrenden Strukturelementen der Formel C) bestehen, worin $R_1$ in 4'- oder 6'-Stellung gebundenes Methyl und $R_2$ Wasserstoff oder $R_1$ Wasserstoff und $R_2$ in 6-Stellung gebundenes Methyl darstellen, $X_1$ Wasserstoff, $X_2$ Wasserstoff oder Methyl und $X_3$ $-CONH_2$, Phenyl, Pyridyl, Pyrrolidyl, —COO—ALkyl—OH mit 2 bis 4 C-Atomen im Alkyl oder —COO—Alkyl mit 1 bis 12 C-Atomen im Alkyl bedeuten und wobei 5 bis 100% der Strukturelemente der Formel B) mit Metallen oder Metallverbindungen komplexiert sind, die keine Alkalimetalle oder Erdalkalimetalle bzw. keine Alkalimetall- oder Erdalkalimetallverbindungen sind.

13. Komplexe von Polymeren nach Anspruch 8 oder 11, worin das Metall ein Metall der Nebengruppen Ib oder VIII, besonders Eisen, Ruthenium, Palladium, Kobalt, Nickel, Platin oder Kupfer ist.

14. Komplexe von Polymeren nach Anspruch 8 oder 11, worin das Metall Ruthenium, Palladium, Platin oder Kupfer ist.

15. Verwendung von mindestens teilweise mit Palladium oder einer Palladiumverbindung komplexierten Polymeren nach Anspruch 8 oder 11 als Katalysatoren für Umvinylierungsreaktionen.

## Claims

1. A compound of the formula I

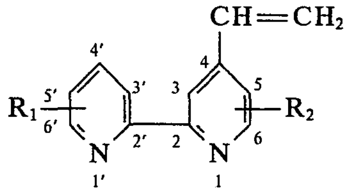

(I)

wherein $R_1$ is hydrogen, $C_{1-6}$alkyl, $C_{1-6}$alkoxy, phenyl or phenoxy and $R_2$ is hydrogen or methyl, or a complex thereof with a metal or metal compound other than an alkali metal or alkaline earth metal, or an alkali metal compound or alkaline earth metal compound.

2. A compound of the formula I according to claim 1, wherein $R_1$ is hydrogen of $C_{1-4}$alkyl bonded in the 4′-position, 5′-position or 6′-position and $R_2$ is hdrogen or methyl, or a complex thereof.

3. A compound of the formula I according to claim 1, wherein $R_1$ is methyl bonded in the 4′-position or 6′-position and $R_2$ is hydrogen, or $R_1$ is hydrogen and $R_2$ is methyl bonded in the 6-position, or a complex thereof.

4. A complex of a compound of the formula I according to claim 1, wherein the metal is a metal of sub-group Ib or VIII, in particular iron, ruthenium, cobalt, nickel, palladium, platinum or copper.

5. A complex of a compound of the formula I according to claim 1, wherein the metal is ruthenium, palladium, platinum or copper.

6. A process for the preparation of a compound of the formula I according to claim 1, which comprises oxidising a compound of the formula II

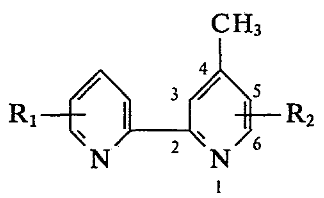

(II)

to a compound of the formula IIIa or IIIb

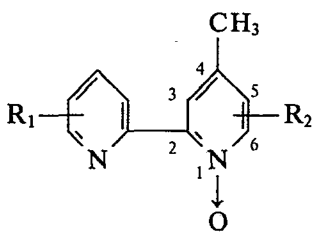

or

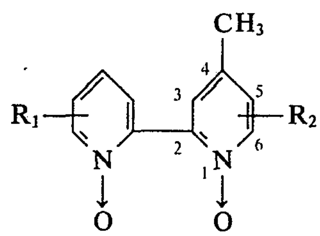

(IIIa) (IIIb)

reacting the compound of the formula IIIa or IIIb with a compound of the formula IV

$(R'CO)_2O$ (IV)

to give a compound of the formula Va or Vb

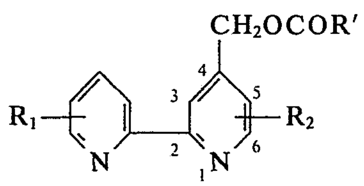

or

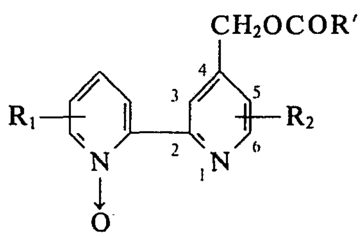

(Va) (Vb)

converting the compound of the formula Vb into a compound of the formula Va, saponifying the compound of the formula Va in the presence of a base to give a compound of the formula VI

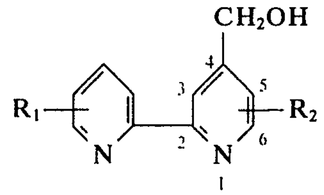

(VI)

converting the compound of the formula VI by treatment with HCl or HBr into the corresponding chloromethyl or bromomethyl compound of the formula VII

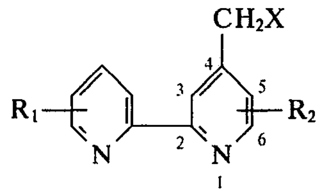

(VII)

reacting the compound of the formula VII with a compound of the formula VIII

$P(R'')_3$ (VIII)

to give a phosphonium salt of the formula IX

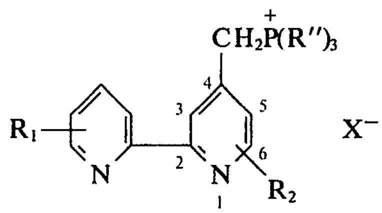

(IX)

reacting the phosphonium salt of the formula IX, in the presence of a base, with formaldehyde to give a compound of the formula I and, of desired, converting a compound of the formula I thus obtained into a complex with a metal or metal compound other an alkali metal or alkaline earth metal, or an alkali metal compound or an alkaline earth metal compound, in which formulae $R_1$ and $R_2$ are as defined for formula I, X is chlorine or bromine, R' is $C_{1-5}$alkyl and each of the substituents R'' independently is $C_{1-5}$alkyl or is phenyl which is unsubstituted or is mono- or disubstituted by $C_{1-4}$alkyl or $C_{1-4}$alkoxy; or of a complex of such a compound.

7. A process for the preparation of a compound of the formula I according to claim 1, which comprises either

a) first treating a compound of the formula II

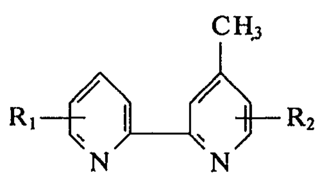

(II)

with a metalating reagent and then converting it with formaldehyde into a compound of the formula X

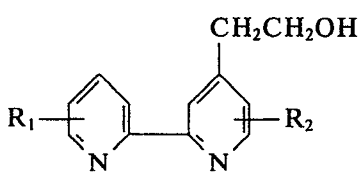

(X)

b) first treating a compound of the formula II with a metalating reagent an then reacting it with a compound of the formula XI

$HalCH_2OCH_2R$ (XI)

to give a compound of the formula XII

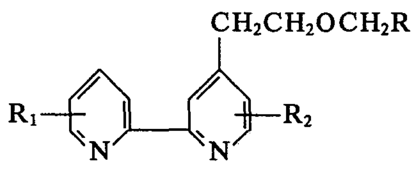

(XII)

hydrogenating the compound of the formula XII to a compound of the formula X and dehydrating the compound of the formula X to a compound of the formula I, or

c) treating a compound of the formula II with a metalating reagent and a compound of the formula XI and heating the resultant reaction mixture and, if desired, converting a compound of the formula I thus obtained into a complex with a metal or metal compound other than an alkali metal or alkaline earth metal, or an alkali metal compound or alkaline earth metal compound, in which formulae $R_1$ and $R_2$ are as defined in claim 1, Hal is a halogen atom and R is phenyl which is unsubstituted or substituted by $C_{1-6}$alkyl or $C_{1-6}$alkoxy; or of a complex of such a sompound.

8. A crosslinked or uncrosslinked polymer, which is obtainable by polymerising 2 to 100 mol% of a compound of the formula I and/or a complex of a compound of the formula I with a metal or metal compound other than an alkali metal or alkaline earth metal, or an alkali metal compound or alkaline earth metal compound, according to claim 1, and 0 to 98 mol% of a compound of the formula A)

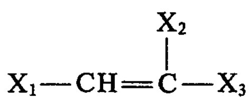

(A)

wherein $X_1$ is hydrogen, $X_2$ is hydrogen, chlorine or methyl and $X_3$ is hydrogen, methyl, chlorine, —CN, —COOH, $-CONH_2$, phenyl, methylphenyl, methoxyphenyl, cyclohexyl, pyridyl, imidazolyl, pyrrolidyl, —COOalkyl having 1 to 12 C atoms in the alkyl moiety, —COOphenyl,

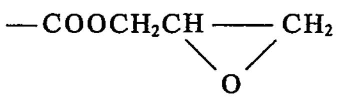

—COOalkyl—OH having 1 to 4 C atoms in the alkyl moiety, —OCOalkyl having 1 to 4 C atoms in the alkyl moiety, —OCOphenyl, —COalkyl having 1 to 3 C atoms in the alkyl moiety, alkoxy having 1 to 20 C atoms or phenoxy, or $X_2$ is hydrogen and $X_1$ and $X_3$ conjointly are an anhydride grouping, a —CO—NR'''—CO— grouping or are each —COOH or —COOalkyl having 1 to 6 atoms in the alkyl moiety, R''' being straight chain or branched $C_{1-18}$alkyl, cyclohexyl or phenyl which can be monosubstituted or disubstituted by $C_{1-16}$alkyl, halogen, cyano, nitro and/or $C_{1-3}$-alkoxy, in the presence of 0 to 60 mol% of a polyunsaturated crosslinking agent and, if desired, converting a complex-forming polymer thus obtained into a polymer which is wholly or partially complexed with a metal or metal compound other than an alkali metal or alkaline earth metal, or an alkali metal compound or alkaline earth metal compound.

9. A crosslinked or uncrosslinked polymer, according to claim 8, which is obtainable by using a compound of the formula (A), wherein $X_1$ is hydrogen, $X_2$ is hydrogen or methyl and $X_3$ is $-CONH_2$, phenyl, pyridyl, pyrrolidyl, —COOalkyl—OH having 2 to 4 C atoms in the alkyl moiety or —COOalkyl having 1 to 12 C atoms in the alkyl moiety, or a complex of such a polymer.

10. A crosslinked polymer according to claim 8, which is obtained by carrying out the polymerisation in the presence of 1 to 30 mol% of a polyunsaturated crosslinking agent, in particular divinylbenzene or divinylpyridine, or a complex of such a polymer.

11. A linear polymer having a mean molecular weight from 1000 to 5 000 000 and consisting of 3—100 mol% of recurring structural elements having the formula B)

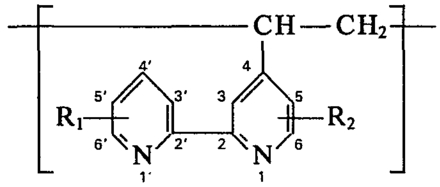

(B)

and/or a complex of such structural elements with metals or metal compounds other than alkali metals or alkaline earth metals, or alkali metal compounds or alkaline earth metal compounds, and of 0 to 97 mol% of recurring structural elements having the formula C)

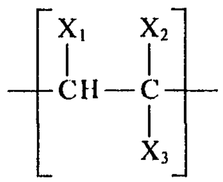

(C)

wherein $R_1$ is hydrogen, $C_{1-6}$alkyl, $C_{1-6}$alkoxy, phenyl or phenoxy and $R_2$ is hydrogen or methyl, with the proviso that one of $R_1$ and $R_2$ is not hydrogen if the $-CH-CH_2-$ group is bonded in the 6-position, $X_1$ is hydrogen, $X_2$ is hydrogen, chlorine or methyl and $X_3$ is hydrogen, methyl, chlorine, $-CN$, $-COOH$, $-CONH_2$, phenyl, methylphenyl, methoxyphenyl, cyclohexyl, pyridyl, imidazolyl, pyrrolidyl, $-COO$alkyl having 1 to 12 C atoms in the alkyl moiety, $-COO$phenyl,

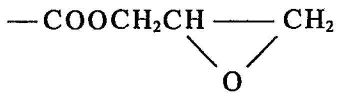

$-COO$alkyl$-OH$ having 1 to 4 C atoms in the alkyl moiety, $-OCO$alkyl having 1 to 4 C atoms in the alkyl moiety, $-OCO$phenyl, $-CO$alkyl having 1 to 3 C atoms in the alkyl moiety, alkoxy having 1 to 20 C atoms or phenoxy, or $X_2$ is hydrogen and $X_1$ and $X_3$ conjointly are an anhydride grouping, a $-CO-NR'''-CO-$ grouping or are each $-COOH$ or $-COO$alkyl having 1 to 6 C atoms in the alkyl moiety, R''' being straight chain or branched $C_{1-18}$alkyl, cyclohexyl or phenyl which can be mono- or disubstituted by $C_{1-6}$alkyl, halogen, cyano, nitro and/or $C_{1-3}$alkoxy.

12. A linear polymer having a mean molecular weight from 2000 to 3 000 000 and/or a complex thereof, according to claim 11, which consists of 3 to 100 mol% of recurring structural elements having the formula B) and/or a complex, according to the definition, of such structural elements having the formula B), and of 0 to 97 mol% of recurring structural elements having the formula C), wherein $R_1$ is methyl bonded in the 4'-position or 6'-position and $R_2$ is hydrogen, or $R_1$ is hydrogen and $R_2$ is methyl bonded in the 6-position, $X_1$ is hydrogen, $X_2$ is hydrogen or methyl and $X_3$ is $-CONH_2$, phenyl, pyridyl, pyrrolidyl, $-COO$alkyl$-OH$ having 2 to 4 C atoms in the alkyl moiety or $-COO$alkyl having 1 to 12 C atoms in the alkyl moiety, 5 to 100 per cent of the structural elements having the formula B) being complexed with a metal or metal compound other than an alkali metal or an alkaline earth metal, or an alkali metal compound or alkaline earth metal compound.

13. A complex of polymer according to either of claim 8 or 11, wherein the metal is a metal of subgroups Ib or VIII, in particular iron, ruthenium, palladium, cobalt, nickel, platinum of copper.

14. A complex of a polymer according to either of claim 8 or 11, wherein the metal is ruthenium, palladium, platinum or copper.

15. Use of a polymer according to either of claims 8 or 11, which is at least partially complexed with palladium or a palladium compound, as a catalyst for transvinylation reactions.

**Revendications**

1. Composés répondant à la formule I:

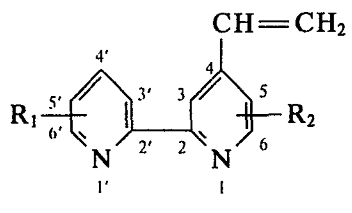

(I)

dans laquelle $R_1$ représente l'hydrogène, un alkyle en $C_1-C_6$, un alcoxy en $C_1-C_6$, un phényle ou un phénoxy et $R_2$ l'hydrogène ou un méthyle, ainsi que les complexes qu'ils forment avec des métaux ou des composés de métaux autres, respectivement, que des métaux alcalins ou alcalinoterreux ou des composés de métaux alcalins ou alcalinoterreux.

2. Composés de formule I et leurs complexes selon la revendication 1, dans lequels $R_1$ représente l'hydrogène ou un radical alkyle en $C_1-C_4$ occupant la position 4', 5' ou 6' et $R_2$ représente l'hydrogène ou un méthyle.

3. Composés de formule I et leurs complexes selon la revendication 1, dans lesquels $R_1$ représente un radical méthyle en position 4' ou en position 6' et $R_2$ l'hydrogène, ou $R_1$ représente l'hydrogène et $R_2$ un radical méthyle en position 6.

4. Complexes de composés de formule I selon la revendication 1, dans lesquels le métal est un métal appartenant à l'un des sous-groupes Ib et VIII, en particulier le fer, le ruthénium, le cobalt, le nickel, le palladium, le platine ou le cuivre.

5. Complexes de composés de formule I selon la revendication 1, dans lesquels le métal est le ruthénium, le palladium, le platine ou le cuivre.

6. Procédé de préparation de composés de formule I ou de leurs complexes selon la revendication 1, procédé caractérisé en ce qu'on oxyde un composé de formule II:

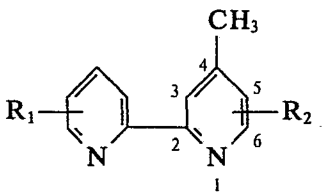

(II)

pour le convertir en un composé de formule IIIa ou IIIb:

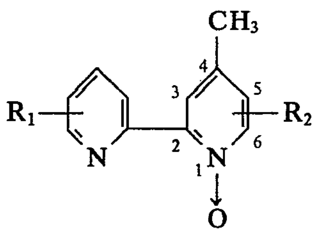

(IIIa)

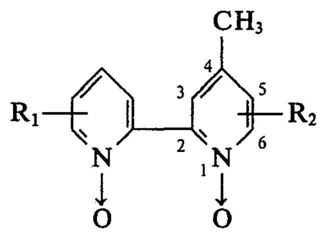

(IIIb)

on fait réagir le composé de formule IIIa ou IIIb avec un composé de formule IV:

$(R'CO)_2O$ (IV)

de manière à obtenir un composé de formule Va ou Vb:

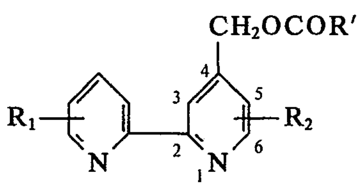

(Va)

et

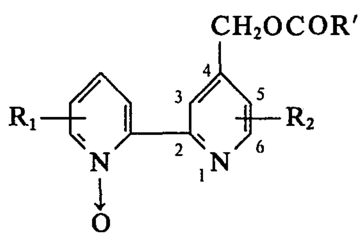

(Vb)

on transforme le composé Vb en un composé de formule Va, on saponifie le composé de formule Va, en présence d'une base, de manière à obtenir un composé de formule VI:

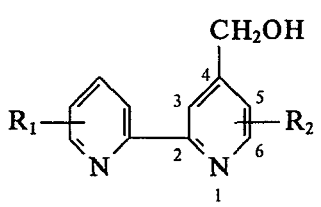

(VI)

on transforme le composé de formule VI, en le traitant par HCl ou HBr, en le composé chlorométhylique ou bromométhylique correspondant de formule VII:

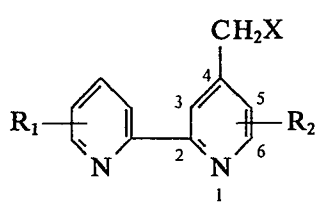

(VII)

on fait réagir le composé de formule VII avec un composé de formule VIII:

$P(R'')_3$ (VIII)

pour obtenir un sel de phosphonium de formule IX:

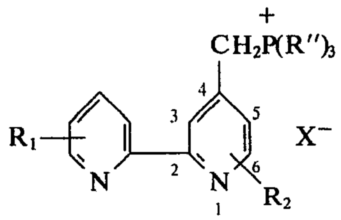

(IX)

on fait réagir le sel de phosphonium de formule IX, en présence d'une base, avec le formaldéhyde de manière à obtenir un composé de formule I et, le cas échéant, on transforme des composés de formule I ainsi obtenus en complexes avec des métaux ou des composés de métaux qui ne sont, respectivement, ni des métaux alcalins ou alcalinoterreux ni des composés de métaux alcalins ou alcalinoterreux; dans les formules précédentes les symboles $R_1$ et $R_2$ ont les significations qui leur ont été données au-dessous de la formule I, X représente le chlore ou le brome, R' représente un alkyle en $C_1-C_5$ et les R'' représentent chacun, indépendamment les uns des autres, un alkyle en $C_1-C_5$ ou un phényle non substitué ou porteur d'un ou de deux substituants pris dans l'ensemble constitué par les alkyles en $C_1-C_4$ et les alcoxy en $C_1-C_4$.

7. Procédé de préparation de composés de formule I ou de complexes de ces composés, selon la revendication 1, procédé caractérisé en ce que ou bien:

a) on traite d'abord un composé de formule II:

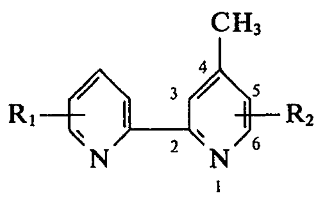

(II)

par un réactif de métallisation, puis on le transforme, au moyen du formaldéhyde, en un composé de formule X:

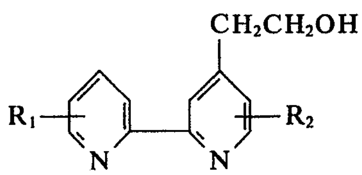

(X)

ou

b) on traite un composé de formule II d'abord par un réactif de métallisation, on le fait ensuite réagir avec un composé de formule XI:

$HalCH_2OCH_2R$ (XI)

de manière à obtenir un composé de formule XII:

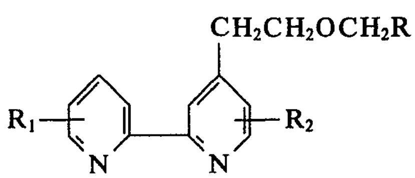

(XII)

puis on hydrogène le composé de formule XII en un composé de formule X,
et on déshydrate le composé de formule X pour le transformer en un composé de formule I,
ou bien,

c) on traite un composé de formule II par un réactif de métallisation et un composé de formule XI et on chauffe le mélange réactionnel obtenu,
et, le cas échant, on transforme les composés de formule I ainsi obtenus en complexes avec des métaux ou des composés des métaux qui ne sont, respectivement, ni des métaux alcalins ou alcalinoterreux ni

des composés de métaux alcalins ou alcalinoterreux; dans les formules précédentes $R_1$ et $R_2$ ont les significations qui leur ont été données à la revendication 1, Hal représente un atome d'halogène et R représente un radical phényle non substitué ou porteur d'un alkyle en $C_1$—$C_6$ ou d'un alcoxy en $C_1$—$C_6$.

8. Polymères éventuellement réticulés que l'on peut obtenir en polymérisant de 2 à 100 % en moles d'un composé de formule I et/ou d'un complexe d'un composé de formule I avec des métaux ou des composés de métaux qui ne sont, respectivement, ni des métaux alcalins ou alcalinoterreux ni des composés de métaux alcalins ou alcalinoterreux, selon la revendication 1, et de 0 à 98 % en moles d'un composé répondant à la formule (A)

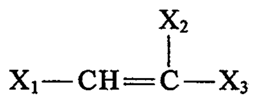

(A)

dans laquelle $X_1$ représente l'hydrogène, $X_2$ l'hydrogène, le chlore ou un méthyle et $X_3$ l'hydrogène, un méthyle, le chlore, $-CN$, $-COOH$, $-CONH_2$, un phényle, un méthylphényle, un méthoxyphényle, un cyclohexyle, un pyridyle, un imidazolyle, un pyrrolidyle, un —COO-alkyl contenant de 1 à 12 atomes de carbone dans l'alkyle, un —COO-phényl,

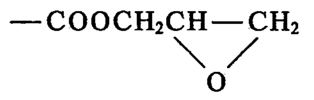

un —COO-alkyl-OH contenant de 1 à 4 atomes de carbone dans l'alkyle, un —OCO-alkyl contenant de 1 à 4 atomes de carbone dans l'alkyle, un —OCO-phényl, un —CO-alkyl contenant de 1 à 3 atomes de carbone dans l'alkyle, un alcoxy contenant de 1 à 20 atomes de carbone ou un phénoxy, ou $X_2$ représente l'hydrogène tandis que $X_1$ et $X_3$ forment ensemble un groupement anhydride ou un groupement $-CO-NR'''-CO-$ ou représentent chacun un radical —COOH ou un radical —COO-alkyl contenant de 1 à 6 atomes de carbone dans l'alkyle, le symbole R''' représentant un alkyle en $C_1$—$C_{18}$ linéaire ou ramifié, un cyclohexyle ou un phényle éventuellement porteur d'un ou de deux substituants pris dans l'ensemble constitué par les alkyles en $C_1$—$C_6$, les halogènes, le cyano, le nitro et les alcoxy en $C_1$—$C_3$, en présence de 0 à 60 % en moles d'un réticulant à plusieurs insaturations, et en transformant éventuellement des polymères complexants ainsi obtenus en polymères qui sont totalement ou partiellement complexés par des métaux ou des composés de métaux autres que, respectivement, des métaux alcalins ou alcalinoterreux ou des composés de métaux alcalins ou alcalinoterreux.

9. Polymères éventuellement reticulés et leurs complexes selon la revendication 8, que l'on peut obtenir en utilisant un composé de formule A dans lequel $X_1$ représente l'hydrogène, $X_2$ l'hydrogène ou un méthyle et $X_3$ un radical $-CONH_2$, phényle, pyridyle, pyrrolidyle, —COO-alkyl-OH à alkyle en $C_2$—$C_4$ ou —COO-alkyl à alkyle en $C_1$—$C_{12}$.

10. Polymères réticulés et leurs complexes, selon la revendication 8, que l'on peut obtenir en effectuant la polymérisation en présence de 1 à 30 % en moles d'un réticulant à plusieurs insaturations, tel que le divinylbenzène ou la divinylpyridine.

11. Polymères linéaires qui ont masse moléculaire moyenne comprise entre 1000 et 5 000 000 et qui sont constitués de 3 à 100 % en moles de motifs répétés de formule B:

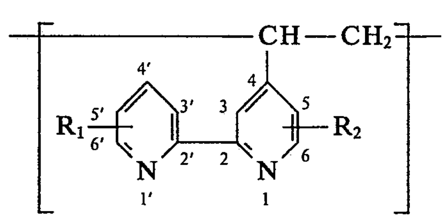

(B)

et/ou de complexes de tels motifs avec des métaux ou des composés de métaux qui ne sont, respectivement, ni des métaux alcalins ou alcalinoterreux ni des composés de métaux alcalins ou alcalinoterreux, et de 0 à 97 % en moles de motifs répétés répondant à la formule C:

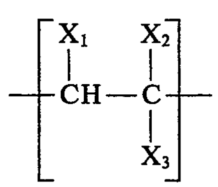

(C)

formules dans lesquelles $R_1$ représente l'hydrogène, un alkyle en $C_1$—$C_6$, un alcoxy en $C_1$—$C_6$, un phényle ou un phénoxy, $R_2$ représente l'hydrogène ou un méthyle avec la condition que l'un des symboles $R_1$ et

$R_2$ soit pas l'hydrogène lorsque le radical $-CH-CH_2-$ se trouve en position 6, $X_1$ représente l'hydrogène, $X_2$ représente l'hydrogène, le chlore ou un méthyle et $X_3$ représente l'hydrogène, un méthyle, le chlore, $-CN$, $-COOH$, $-CONH_2$, un méthyle, un méthylphényle, un méthoxyphényle, un cyclohexyle, un pyridyle, un imidazolyle, un pyrrolidyle, un radical $-$COO-alkyl dont l'alkyle contient de 1 à 12 atomes de carbone, un radical $-$COO-phényl,

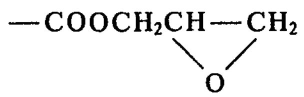

un radical $-$COO-alkyl-OH dont l'alkyle contient de 1 à 4 atomes de carbone, un radical $-$OCO-alkyle dont l'alkyle contient de 1 à 4 atomes de carbone, un radical $-$OCO-phényl, un radical $-$CO-alkyl dont l'alkyle contient de 1 à 3 atomes de carbone, un alcoxy en $C_1-C_{20}$ ou un phénoxy, ou $X_2$ représente l'hydrogène tandis que $X_1$ et $X_3$ forment ensemble un groupement anhydride ou un groupement $-CO-NR'''-CO-$ ou représentent chacun un radical $-COOH$ ou un radical $-$COO-alkyl dont l'alkyle contient de 1 à 6 atomes de carbone, le symbole R''' représentant un alkyle en $C_1-C_{18}$ linéaire ou ramifié, un cyclohexyle ou un phényle éventuellement porteur d'un ou de deux substituants pris dans l'ensemble constitué par les alkyles en $C_1-C_6$, les halogènes, le cyano, le nitro et les alcoxy en $C_1-C_3$.

12. Polymères linéaires de masse moléculaire moyenne comprise entre 2000 et 3 000 000 et/ou leurs complexes selon la revendication 11, qui sont constitués de 3 à 100 % en moles de motifs répétés de formule B et/ou de complexes, conformes à la définition, de tels motifs de formule B, et de 0 à 97 % en moles de motifs répétés de formule C, motifs dans lesquels $R_1$ représente un méthyle en position 4' ou 6' et $R_2$ l'hydrogène ou $R_1$ représente l'hydrogène et $R_2$ un méthyle en position 6, $X_1$ représente l'hydrogène, $X_2$ représente l'hydrogène ou un méthyle et $X_3$ représente $-CONH_2$, un phényle, un pyridyle, un pyrrolidyle, un radical $-$COO-alkyl-OH contenant de 2 à 4 atomes de carbone dans son alkyle ou un radical $-$COO-alkyl dont l'alkyle contient de 1 à 12 atomes de carbone, de 5 à 100 % des motifs de formule B étant complexés par des métaux ou des composés de métaux qui ne sont, respectivement, ni des métaux alcalins ou alcalinoterreux ni des composés de métaux alcalins ou alcalinoterreux.

13. Complexes de polymères selon l'une des revendications 8 et 11, dans lesquels le métal est un métal appartenant à l'un des sous-groupes Ib et VIII, en particulier, le fer, le ruthénium, le palladium, le cobalt, le nickel, le platine ou le cuivre.

14. Complexes de polymères selon l'une des revendications 8 et 11, dans lesquels le métal est le ruthénium, le palladium, le platine ou le cuivre.

15. Application de polymères selon l'une des revendications 8 et 11, au moins partiellement complexés par du palladium ou un composé du palladium, comme catalyseurs pour des réactions de trans-vinylation.